# EUROPEAN PATENT SPECIFICATION

(11) **EP 4 072 438 B1**
(45) Date of publication and mention of the grant of the patent: **29.04.2026**
(21) Application number: 21819974.3
(22) Date of filing: 29.11.2021
(51) Int. Cl.: A61B 17/072, A61B 90/00

(54) **DUAL-SIDED REINFORCED RELOAD FOR SURGICAL INSTRUMENTS**
DOPPELSEITIGE VERSTÄRKTE NACHLADUNG FÜR CHIRURGISCHE INSTRUMENTE
RECHARGE RENFORCÉE DOUBLE FACE POUR INSTRUMENTS CHIRURGICAUX

(30) Priority: 02.12.2020 US 202017109589; 02.12.2020 US 202017109627
(43) Date of publication of application: 19.10.2022
(73) Proprietor: Cilag GmbH International, 6300 Zug (CH)
(72) Inventor: SHELTON, IV, Frederick E., Cincinnati, Ohio 45242 (US); FIEBIG, Kevin M., Cincinnati, Ohio 45242 (US); BRADY, John E., Cincinnati, Ohio 45242 (US); WARD, Andréas N., Cincinnati, Ohio 45242 (US); MORGAN, Nicholas M., Cincinnati, Ohio 45242 (US); HARBACH, Diana M., Cincinnati, Ohio 45242 (US); HAMANN, David L., Cincinnati, Ohio 45242 (US)
(74) Representative: Carpmaels & Ransford LLP
(86) International application number: PCT/IB2021/061051
(87) International publication number: WO 2022/118161

(56) References cited:
- EP-A2- 2 644 119
- US-A1- 2018 235 626

## Description

### BACKGROUND

The present invention relates to surgical instruments and, in various arrangements, to surgical stapling and cutting instruments and staple cartridges for use therewith that are designed to staple and cut tissue.

EP2644119A2 discusses a tissue thickness compensator that can comprise a film body formed from a continuous extruded shape and, in addition, a fibrous medicament core.

US2018/0235626A1 discusses methods for applying one or more adjuncts to tissue. The method can include positioning an adjunct, e.g., using an adhesive, on one of first and second jaws of an end effector of a surgical stapler. An attachment mechanism on the adjunct can prevent stretching of at least a portion of the adjunct. The adjunct can be maintained on the at least one jaw in a first state in which the adjunct is at least partially stretched over the at least one jaw, and actuation of the surgical stapler can cause the adjunct to transition from the first state to a second state such that the adjunct in the second state at least partially separates from the at least one jaw.

### SUMMARY

In various embodiments, a buttress applier cartridge configured to interface with an end effector of a surgical stapling instrument is disclosed. The buttress applier cartridge includes a buttress loading zone configured to interface with an anvil of the end effector. The buttress loading zone includes a buttress layer, a suture supporting the buttress layer, and a loading assembly for securing the suture to the anvil as the anvil approaches the buttress layer.

### BRIEF DESCRIPTION OF THE DRAWINGS

Various features of the embodiments described herein, together with advantages thereof, may be understood in accordance with the following description taken in conjunction with the accompanying drawings as follows:
FIG. 1 illustrates a perspective view of an exemplary articulating surgical stapling instrument.
FIG. 2 illustrates a perspective view of an end effector of the instrument of FIG. 1, with the end effector in an open configuration.
FIG. 3 illustrates an exploded perspective view of the end effector of FIG. 2.
FIG. 4 illustrates a perspective view of an exemplary upper buttress and an exemplary lower buttress, each of which may be applied to the end effector of FIG. 2.
FIG. 5 illustrates a buttress applier cartridge, according to at least one aspect of the present disclosure.
FIG. 6 illustrates the buttress applier cartridge of FIG. 1 receiving an end effector, according to at least one aspect of the present disclosure.
FIG. 7 illustrates an anvil prior to receiving a suture from a buttress applier cartridge, according to at least one aspect of the present disclosure.
FIG. 8 illustrates the buttress applier cartridge of FIG. 1 interfacing with an end effector, according to at least one aspect of the present disclosure.
FIG. 9 illustrates an anvil after receiving a suture from a buttress applier cartridge, according to at least one aspect of the present disclosure.
FIG. 10 illustrates a buttress applier cartridge, according to at least one aspect of the present disclosure
FIG. 11 illustrates a suture grabber, according to at least one aspect of the present disclosure.
FIG. 12 illustrates a side view of the suture grabber of FIG. 11, according to at least one aspect of the present disclosure.
FIG. 13 illustrates a suture grabber, according to at least one aspect of the present disclosure.
FIG. 14 illustrates a suture grabber, according to at least one aspect of the present disclosure.
FIG. 15 illustrates a side view of the suture grabber of FIG. 15, according to at least one aspect of the present disclosure.
FIG. 16 illustrates a suture grabber, according to at least one aspect of the present disclosure.
FIG. 17 illustrates an embodiment for securing a buttress to an anvil, according to at least one aspect of the present disclosure.
FIG. 18 illustrates a cross-section view of FIG. 17, according to at least one aspect of the present disclosure.
FIG. 19 illustrates a buttress applier cartridge, according to at least one aspect of the present disclosure.
FIG. 20 illustrates the buttress applier cartridge of FIG. 19 before and after interfacing with an end effector, according to at least one aspect of the present disclosure.
FIG. 21 illustrates a buttress applier cartridge, according to at least one aspect of the present disclosure.
FIG. 22 illustrates a buttress applier cartridge, according to at least one aspect of the present disclosure.
FIG. 23 illustrates a buttress applier cartridge, according to at least one aspect of the present disclosure.
FIG. 24 illustrates the buttress applier cartridge of FIG. 23 when interfacing with an anvil, according to at least one aspect of the present disclosure.
FIG. 25 illustrates a buttress assembly, according to at least one aspect of the present disclosure.
FIG. 26 illustrates the buttress assembly of FIG. 21 being removed from an anvil after a surgical stapling procedure, according to at least one aspect of the present disclosure.
FIG. 27 illustrates a buttress assembly interfacing with an anvil, according to at least one aspect of the present disclosure.
FIG. 28 illustrates a portion of the buttress assembly of FIG. 27 coupled to an anvil, according to at least one aspect of the present disclosure.
FIG. 29 illustrates a portion of the buttress assembly of FIG. 27 interfacing with a knife member, according to at least one aspect of the present disclosure.
FIG. 30 illustrates an anvil interfacing with a buttress layer, according to at least aspect of the present disclose
FIG. 31 illustrates a suture receiver, according to at least one aspect of the present disclosure.
FIG. 32 illustrates the anvil and buttress layer of FIG. 30 coupled together, according to at least one aspect of the present disclosure.
FIG. 33 illustrates the anvil of FIG. 30 decoupled from the buttress layer, according to at least one aspect of the present disclosure.
FIG. 34 illustrates a side view of a lockout mechanism, according to at least one aspect of the present disclosure.
FIG. 35 illustrates a lockout mechanism in an unlocked state, according to at least one aspect of the present disclosure.
FIG. 36 illustrates a lockout mechanism in a lockout state, according to at least one aspect of the present disclosure.
FIG. 37 illustrates a suture applier, according to at least one aspect of the present disclosure.
FIG. 38 illustrates the suture applier of FIG. 37 in an open position interfacing with an end effector, according to at least one aspect of the present disclosure.
FIG. 39 illustrates a top view of FIG. 38, according to at least one aspect of the present disclosure.
FIG. 40 illustrates the suture applier of FIG. 37 in a closed position interfacing with an end effector, according to at least one aspect of the present disclosure.
FIG. 41 illustrates the suture applier of FIG. 37 moving to the open position after closing onto the end effector, according to at least one aspect of the present disclosure.
FIG. 42 illustrates a plunger assembly of the suture applier of FIG. 37, according to at least one aspect of the present disclosure.
FIG. 43 illustrates an anvil, according to at least one aspect of the present disclosure.
FIG. 44 illustrates a suture assembly, according to at least one aspect of the present disclosure.
FIG. 45 illustrates a buttress cartridge usable with the anvil of FIG. 43, according to at least one aspect of the present disclosure.
FIG. 46 illustrates an arm of the buttress cartridge of FIG. 45 contacting a cam lock of the anvil of FIG. 43, according to at least one aspect of the present disclosure.
FIG. 47 illustrates a hooked shaped needle, according to at least one aspect of the present disclosure.
FIG. 48 illustrates a detailed view of a cam lock, according to at least one aspect of the present disclosure.
FIG. 49 illustrates a buttress applier cartridge, according to at least one aspect of the present disclosure.
FIG. 50 illustrates a zoomed view of the buttress applier cartridge of FIG. 49, according to at least one aspect of the present disclosure.
FIG. 51 illustrates a buttress assembly, according to at least one aspect of the present disclosure.
FIG. 52 illustrates a cross-sectional view of the buttress assembly of FIG. 51, according to at least one aspect of the present disclosure.
FIG. 53 illustrates an anvil interfacing with a proximal-most suture clamp of a buttress applier cartridge, according to at least one aspect of the present disclosure.
FIG. 54 illustrates a detailed, top view of a suture clamp, according to at least one aspect of the present disclosure.
FIG. 55 illustrates an anvil, according to at least one aspect of the present disclosure.
FIG. 56 illustrates an anvil interfacing with a buttress assembly, according to at least one aspect of the present disclosure.
FIG. 57 illustrates a cross-sectional view of the buttress assembly of FIG. 56 positioned within the anvil of FIG. 56, according to at least one aspect of the present disclosure.
FIG. 58 illustrates a tissue contacting surface of an anvil, according to at least one aspect of the present disclosure.
FIG. 59 illustrates an outer surface of the anvil of FIG. 58, according to at least one aspect of the present disclosure.
FIG. 60 illustrates an isometric view of a suture lock, according to at least one aspect of the present disclosure.
FIG. 61 illustrates a zoomed view of the suture lock of FIG. 59, according to at least one aspect of the present disclosure.
FIG. 62 illustrates a buttress layer, according to at least one aspect of the present disclosure.
FIG. 63 illustrates the buttress layer of FIG. 62 interfacing with the anvil of FIG. 59, according to at least one aspect of the present disclosure.
FIG. 64 illustrates distal-most suture legs of the buttress layer wrapping around the suture lock, according to at least one aspect of the present disclosure.
FIG. 65 illustrates proximal-most suture legs of the buttress layer wrapping around the suture lock, according to at least one aspect of the present disclosure.
FIG. 66 illustrates the buttress layer being released from the anvil, according to at least one aspect of the present disclosure.

Corresponding reference characters indicate corresponding parts throughout the several views. The exemplifications set out herein illustrate certain embodiments of the invention, in one form, and such exemplifications are not to be construed as limiting the scope of the invention in any manner.

### DETAILED DESCRIPTION

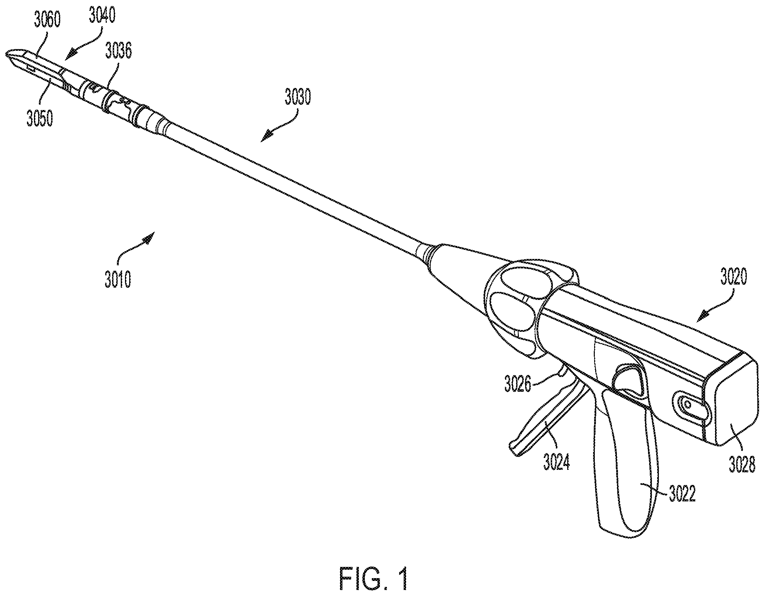

FIG. 1 depicts an exemplary surgical stapling and severing instrument 3010 that includes a handle assembly 3020, a shaft assembly 3030, and an end effector 3040. End effector 3040 and the distal portion of shaft assembly 3030 are sized for insertion, in a nonarticulated state as depicted in FIG. 1, through a trocar cannula to a surgical site in a patient for performing a surgical procedure. By way of example only, such a trocar may be inserted in a patient's abdomen, between two of the patient's ribs, or elsewhere. In some settings, instrument 3010 is used without a trocar. For instance, end effector 3040 and the distal portion of shaft assembly 3030 may be inserted directly through a thoracotomy or other type of incision. It should be understood that terms such as "proximal" and "distal" are used herein with reference to a clinician gripping handle assembly 3020 of instrument 3010. Thus, end effector 3040 is distal with respect to the more proximal handle assembly 3020. It will be further appreciated that for convenience and clarity, spatial terms such as "vertical" and "horizontal" are used herein with respect to the drawings. However, surgical instruments are used in many orientations and positions, and these terms are not intended to be limiting and absolute.

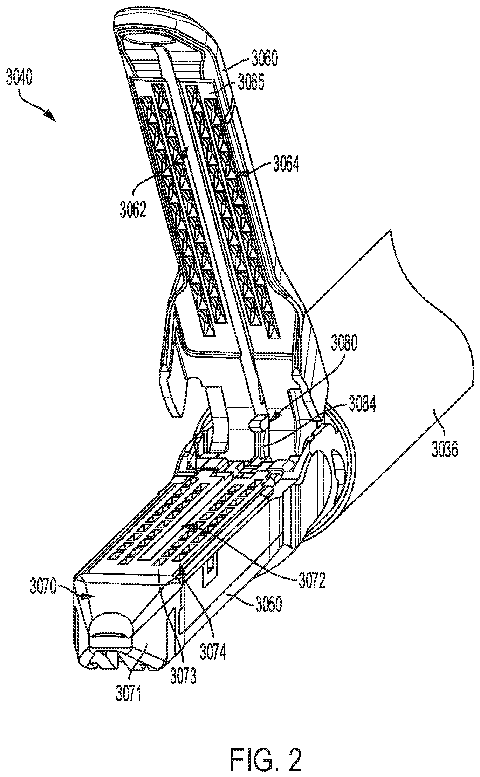

As also shown in FIGS. 1-3, end effector 3040 of the present example includes a lower jaw 3050 and a pivotable anvil 3060. Anvil 3060 includes a pair of integral, outwardly extending pins 3066 that are disposed in corresponding curved slots 3054 of lower jaw 3050. Anvil 3060 is pivotable toward and away from lower jaw 3050 between an open position (shown in FIG. 2) and a closed position (shown in FIG. 1). Use of the term "pivotable" (and similar terms with "pivot" as a base) should not be read as necessarily requiring pivotal movement about a fixed axis. For instance, in the present example, anvil 3060 pivots about an axis that is defined by pins 3066, which slide along curved slots 3054 of lower jaw 3050 as anvil 3060 moves toward lower jaw 3050. In such versions, the pivot axis translates along the path defined by slots 3054 while anvil 3060 simultaneously pivots about that axis. In addition or in the alternative, the pivot axis may slide along slots 3054 first, with anvil 3060 then pivoting about the pivot axis after the pivot axis has slid a certain distance along the slots 3054. It should be understood that such sliding/translating pivotal movement is encompassed within terms such as "pivot," "pivots," "pivotal," "pivotable," "pivoting," and the like. Of course, some versions may provide pivotal movement of anvil 3060 about an axis that remains fixed and does not translate within a slot or channel, etc.

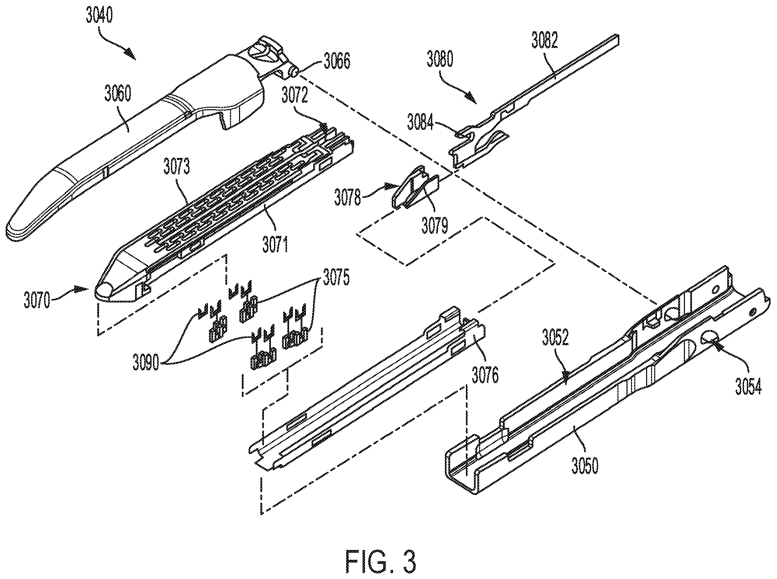

As best seen in FIG. 3, lower jaw 3050 of the present example defines a channel 3052 that is configured to receive a staple cartridge 3070. Staple cartridge 3070 may be inserted into channel 3052, end effector 3040 may be actuated, and then staple cartridge 3070 may be removed and replaced with another staple cartridge 3070. Lower jaw 3050 thus releasably retains staple cartridge 3070 in alignment with anvil 3060 for actuation of end effector 3040. In some versions, lower jaw 3050 is constructed in accordance with at least some of the teachings of U.S. Patent Application Publication No. 2014/0239044, entitled INSTALLATION FEATURES FOR SURGICAL INSTRUMENT END EFFECTOR CARTRIDGE, published August 28, 2014, issued as U.S. Patent No. 9,808,248 on November 30, 2016. Other suitable forms that lower jaw 3050 may take will be apparent to those of ordinary skill in the art in view of the teachings herein.

As best seen in FIGS. 2 and 3, staple cartridge 3070 of the present example comprises a cartridge body 3071 and a tray 3076 secured to the underside of cartridge body 3071. The upper side of cartridge body 3071 presents a deck 3073, against which tissue may be compressed when anvil 3060 is in a closed position. Cartridge body 3071 further defines a longitudinally extending channel 3072 and a plurality of staple pockets 3074. A staple 3090 is positioned in each staple pocket 3074. A staple driver 3075 is also positioned in each staple pocket 3074, underneath a corresponding staple 3090, and above tray 3076. As will be described in greater detail below, staple drivers 3075 are operable to translate upwardly in staple pockets 3074 to thereby drive staples 3090 upwardly through staple pockets 3074 and into engagement with anvil 3060. Staple drivers 3075 are driven upwardly by a wedge sled 3078, which is captured between cartridge body 3071 and tray 3076, and which translates longitudinally through cartridge body 3071.

Wedge sled 3078 includes a pair of obliquely angled cam surfaces 3079, which are configured to engage staple drivers 3075 and thereby drive staple drivers 3075 upwardly as wedge sled 3078 translates longitudinally through cartridge 3070. For instance, when wedge sled 3078 is in a proximal position, staple drivers 3075 are in downward positions and staples 3090 are located in staple pockets 3074. As wedge sled 3078 is driven to the distal position by a translating knife member 3080, wedge sled 3078 drives staple drivers 3075 upwardly, thereby driving staples 3090 out of staple pockets 3074 and into staple forming pockets 3064 that are formed in the underside 3065 of anvil 3060. Thus, staple drivers 3075 translate along a vertical dimension as wedge sled 3078 translates along a horizontal dimension.

In some versions, staple cartridge 3070 is constructed and operable in accordance with at least some of the teachings of U.S. Patent Application Publication No. 2014/0239042, entitled INTEGRATED TISSUE POSITIONING AND JAW ALIGNMENT FEATURES FOR SURGICAL STAPLER, published August 28, 2014, issued as U.S. Patent No. 9,517,065 on December 13, 2016. In addition or in the alternative, staple cartridge 3070 may be constructed and operable in accordance with at least some of the teachings of U.S. Patent Application Publication No. 2014/0239044, entitled INSTALLATION FEATURES FOR SURGICAL INSTRUMENT END EFFECTOR CARTRIDGE, published August 28, 2014, issued as U.S. Patent No. 9,808,248 on November 7, 2017. Other suitable forms that staple cartridge 3070 may take will be apparent to those of ordinary skill in the art in view of the teachings herein.

As best seen in FIG. 2, anvil 3060 of the present example comprises a longitudinally extending channel 3062 and a plurality of staple forming pockets 3064. Channel 3062 is configured to align with channel 3072 of staple cartridge 3070 when anvil 3060 is in a closed position. Each staple forming pocket 3064 is positioned to lie over a corresponding staple pocket 3074 of staple cartridge 3070 when anvil 3060 is in a closed position. Staple forming pockets 3064 are configured to deform the legs of staples 3090 when staples 3090 are driven through tissue and into anvil 3060. In particular, staple forming pockets 3064 are configured to bend the legs of staples 3090 to secure the formed staples 3090 in the tissue. Anvil 3060 may be constructed in accordance with at least some of the teachings of U.S. Patent Application Publication No. 2014/0239042, entitled INTEGRATED TISSUE POSITIONING AND JAW ALIGNMENT FEATURES FOR SURGICAL STAPLER, published August 28, 2014, issued as U.S. Patent No. 9,517,065 on December 13, 2016; at least some of the teachings of U.S. Patent Application Publication No. 2014/0239036, entitled JAW CLOSURE FEATURE FOR END EFFECTOR OF SURGICAL INSTRUMENT, published August 28, 2014, issued as U.S. Patent No. 9,839,421 on December 12, 2017; and/or at least some of the teachings of U.S. Patent Application Publication No. 2014/0239037, entitled STAPLE FORMING FEATURES FOR SURGICAL STAPLING INSTRUMENT, published August 28, 2014, issued as U.S. Patent No. 10,092,292 on October 9, 2018. Other suitable forms that anvil 3060 may take will be apparent to those of ordinary skill in the art in view of the teachings herein.

In the present example, a knife member 3080 is configured to translate through end effector 3040. As best seen in FIG. 3, knife member 3080 is secured to the distal end of a firing beam 3082, which extends through a portion of shaft assembly 3030. As best seen in FIG. 2, knife member 3080 is positioned in channels 3062, 3072 of anvil 3060 and staple cartridge 3070. Knife member 3080 includes a distally presented cutting edge 3084 that is configured to sever tissue that is compressed between anvil 3060 and deck 3073 of staple cartridge 3070 as knife member 3080 translates distally through end effector 3040. As noted above, knife member 3080 also drives wedge sled 3078 distally as knife member 3080 translates distally through end effector 3040, thereby driving staples 3090 through tissue and against anvil 3060 into formation.

In the present example, anvil 3060 is driven toward lower jaw 3050 by advancing closure ring 3036 distally relative to end effector 3040. Closure ring 3036 cooperates with anvil 3060 through a camming action to drive anvil 3060 toward lower jaw 3050 in response to distal translation of closure ring 3036 relative to end effector 3040. Similarly, closure ring 3036 may cooperate with anvil 3060 to open anvil 3060 away from lower jaw 3050 in response to proximal translation of closure ring 3036 relative to end effector 3040. By way of example only, closure ring 3036 and anvil 3060 may interact in accordance with at least some of the teachings of U.S. Patent Application Publication No. 2014/0239036, entitled JAW CLOSURE FEATURE FOR END EFFECTOR OF SURGICAL INSTRUMENT, published August 28, 2014, issued as U.S. Patent No. 9,839,421 on December 12, 2017; and/or in accordance with at least some of the teachings of U.S. Patent Application Serial No. 14/314,108, entitled JAW OPENING FEATURE FOR SURGICAL STAPLER, filed on June 25, 2014, published as U.S. Patent Application Publication No. 2015/0374373 on December 31, 2015.

Handle assembly 3020 includes a pistol grip 3022 and a closure trigger 3024. As noted above, anvil 3060 is closed toward lower jaw 3050 in response to distal advancement of closure ring 3036. In the present example, closure trigger 3024 is pivotable toward pistol grip 3022 to drive closure tube 3032 and closure ring 3036 distally. Various suitable components that may be used to convert pivotal movement of closure trigger 3024 toward pistol grip 3022 into distal translation of closure tube 3032 and closure ring 3036 relative to handle assembly 3020 will be apparent to those of ordinary skill in the art in view of the teachings herein.

Also in the present example, instrument 3010 provides motorized control of firing beam 3082. In particular, instrument 3010 includes motorized components that are configured to drive firing beam 3082 distally in response to pivoting of firing trigger 3026 toward pistol grip 3022. In some versions, a motor (not shown) is contained in pistol grip 3022 and receives power from battery pack 3028. This motor is coupled with a transmission assembly (not shown) that converts rotary motion of a drive shaft of the motor into linear translation of firing beam 3082. By way of example only, the features that are operable to provide motorized actuation of firing beam 3082 may be configured and operable in accordance with at least some of the teachings of U.S. Patent No. 8,210,411, entitled MOTOR-DRIVEN SURGICAL INSTRUMENT, issued July 3, 2012; U.S. Patent No. 8,453,914, entitled MOTOR-DRIVEN SURGICAL CUTTING INSTRUMENT WITH ELECTRIC ACTUATOR DIRECTIONAL CONTROL ASSEMBLY, issued June 4, 2013; and/or U.S. Patent Application Serial No. 14/226,142, entitled SURGICAL INSTRUMENT COMPRISING A SENSOR SYSTEM, filed March 26, 2014, issued as U.S. Patent No. 9,913,642 on March 13, 2018.

Additional details regarding the exemplary surgical stapling and severing instrument 3010 can be found in U.S. Patent No. 10,342,542.

In some instances, it may be desirable to equip end effector 3040 with a buttress material to reinforce the mechanical fastening of tissue provided by staples 3090. Such a buttress may prevent the applied staples 3090 from pulling through the tissue and may otherwise reduce a risk of tissue tearing at or near the site of applied staples 3090. In addition to or as an alternative to providing structural support and integrity to a line of staples 3090, a buttress may provide various other kinds of effects such as spacing or gap-filling, administration of therapeutic agents, and/or other effects. In some instances, a buttress may be provided on deck 3073 of staple cartridge 3070. In some other instances, a buttress may be provided on the surface of anvil 3060 that faces staple cartridge 3070. It should also be understood that a first buttress may be provided on deck 3073 of staple cartridge 3070 while a second buttress is provided on anvil 3060 of the same end effector 3040. Various examples of forms that a buttress may take will be described in greater detail below. Various ways in which a buttress may be secured to a staple cartridge 3070 or an anvil 3060 will also be described in greater detail below.

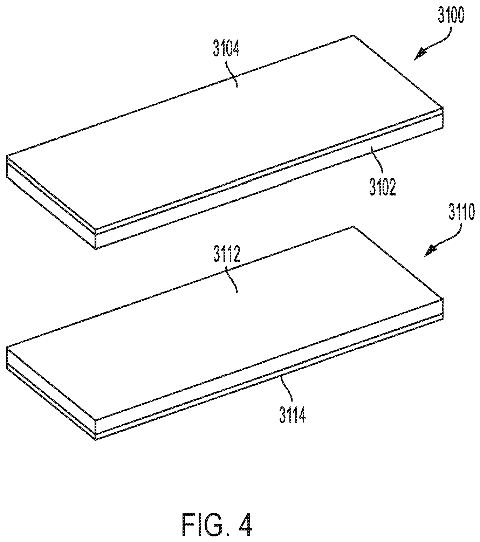

FIG. 4 shows an exemplary pair of buttress assemblies 3100, 3110 with a basic composition. Buttress assembly 3100 of this example comprises a buttress body 3102 and an upper adhesive layer 3104. Similarly, buttress assembly 3110 comprises a buttress body 3112 and a lower adhesive layer 3114. In the present example, each buttress body 3102, 3112 comprises a strong yet flexible material configured to structurally support a line of staples 3090. By way of example only, each buttress body 3102, 3112 may comprise a woven mesh of polyglactin 910 material by Ethicon, Inc. of Somerville, N.J. Alternatively, any other suitable materials or combinations of materials may be used in addition to or as an alternative to polyglactin 910 material to form each buttress body 3102, 3112. Each buttress body 3102, 3112 may take any other suitable form and may be constructed of any other suitable material(s). By way of further example only, each buttress body 3102, 3112 may comprise one or more of the following: NEOVEIL absorbable PGA felt by Gunze Limited, of Kyoto, Japan; SEAMGUARD polyglycolic acid:trimethylene carbonate (PGA:TMC) reinforcement material by W.L. Gore & Associates, Inc., of Flagstaff, Ariz.; PERI-STRIPS DRY with VERITAS Collagen Matrix (PSDV) reinforcement material, by Baxter Healthcare Corporation of Deerfield, Ill.; BIODESIGN biologic graft material by Cook Medical, Bloomington, Ind.; and/or SURGICEL NU-KNIT hemostat material by Ethicon, Inc. of Somerville, N.J. Still other suitable materials that may be used to form each buttress body 3102, 3112 will be apparent to those of ordinary skill in the art in view of the teachings herein.

In addition or in the alternative, each buttress body 3102, 3112 may comprise a material including, for example, a hemostatic agent such as fibrin to assist in coagulating blood and reduce bleeding at the severed and/or stapled surgical site along tissue. As another merely illustrative example, each buttress body 3102, 3112 may comprise other adjuncts or hemostatic agents such as thrombin may be used such that each buttress body 3102, 3112 may assist to coagulate blood and reduce the amount of bleeding at the surgical site. Other adjuncts or reagents that may be incorporated into each buttress body 3102, 3112 may further include but are not limited to medical fluid or matrix components. Merely illustrative examples of materials that may be used to form each buttress body 3102, 3112, as well as materials that may be otherwise incorporated into each buttress body 3102, 3112, are disclosed in U.S. Patent Application Serial No. 14/667,842, entitled METHOD OF APPLYING A BUTTRESS TO A SURGICAL STAPLER, filed March 25, 2015, published as U.S. Patent Application Publication No. 2016/0278774 on September 29, 2016. Alternatively, any other suitable materials may be used.

By way of further example only, each buttress body 3102, 3112 may be constructed in accordance with at least some of the teachings of U.S. Patent Application Publication No. 2012/0241493, entitled TISSUE THICKNESS COMPENSATOR COMPRISING CONTROLLED RELEASE AND EXPANSION, published September 27, 2012, issued as U.S. Patent No. 10,123,798 on November 13, 2018; U.S. Patent Application Publication No. 2013/0068816, entitled SURGICAL INSTRUMENT AND BUTTRESS MATERIAL, published March. 21, 2013, now abandoned; U.S. Patent Application Publication No. 2013/0062391, entitled SURGICAL INSTRUMENT WITH FLUID FILLABLE BUTTRESS, published March 14, 2013, issued as U.S. Patent No. 9,999,408 on June 19, 2018; U.S. Patent Application Publication No. 2013/0068820, entitled FIBRIN PAD MATRIX WITH SUSPENDED HEAT ACTIVATED BEADS OF ADHESIVE, published March 21, 2013, issued as U.S. Patent No. 8,814,025 on August 26, 2014; U.S. Patent Application Publication No. 2013/0082086, entitled ATTACHMENT OF SURGICAL STAPLE BUTTRESS TO CARTRIDGE, published April 4, 2013, issued as U.S. Patent No. 8,899,464 on December 2, 2014; U.S. Patent Application Publication No. 2013/0037596, entitled DEVICE FOR APPLYING ADJUNCT IN ENDOSCOPIC PROCEDURE, published February 14, 2013, issued as U.S. Patent No. 9,492,170 on November 15, 2016; U.S. Patent Application Publication No. 2013/0062393, entitled RESISTIVE HEATED SURGICAL STAPLE CARTRIDGE WITH PHASE CHANGE SEALANT, published March 14, 2013, issued as U.S. Patent No. 8,998,060 on April 7, 2015; U.S. Patent Application Publication No. 2013/0075446, entitled SURGICAL STAPLE ASSEMBLY WITH HEMOSTATIC FEATURE, published March 28, 2013, issued as U.S. Patent No. 9,393,018 on July 19, 2016; U.S. Patent Application Publication No. 2013/0062394, entitled SURGICAL STAPLE CARTRIDGE WITH SELF-DISPENSING STAPLE BUTTRESS, published March 14, 2013, issued as U.S. Patent No. 9,101,359 on August 11, 2015; U.S. Patent Application Publication No. 2013/0075445, entitled ANVIL CARTRIDGE FOR SURGICAL FASTENING DEVICE, published March 28, 2013, issued as U.S. Patent No. 9,198,644 on December 1, 2015; U.S. Patent Application Publication No. 2013/0075447, entitled ADJUNCT THERAPY FOR APPLYING HEMOSTATIC AGENT, published March 28, 2013, now abandoned; U.S. Patent Application Publication No. 2013/0256367, entitled TISSUE THICKNESS COMPENSATOR COMPRISING A PLURALITY OF MEDICAMENTS, published October 3, 2013, issued as U.S. Patent No. 9,211,120 on December 15, 2015; U.S. Patent Application Serial No. 14/300,954, entitled ADJUNCT MATERIALS AND METHODS OF USING SAME IN SURGICAL METHODS FOR TISSUE SEALING, filed June 10, 2014, issued as U.S. Patent No. 10,172,611 on Jan. 8, 2019; U.S. Patent Application Serial No. 14/827,856, entitled IMPLANTABLE LAYERS FOR A SURGICAL INSTRUMENT, filed August 17, 2015, published as U.S. Patent Application Publication No. 2017/0049444 on February 23, 2017; U.S. Patent Application Serial No. 14/840,613, entitled DRUG ELUTING ADJUNCTS AND METHODS OF USING DRUG ELUTING ADJUNCTS, filed August 31, 2015, published as U.S. Patent Application Publication No. 2017/0055986 on March 2, 2017; U.S. Patent Application Serial No. 14/871,071, entitled COMPRESSIBLE ADJUNCT WITH CROSSING SPACER FIBERS, filed September 30, 2015, published as U.S. Patent Application Publication No. 2017/0086837 on March 30, 2017; and/or U.S. Patent Application Serial No. 14/871,131, entitled METHOD FOR APPLYING AN IMPLANTABLE LAYER TO A FASTENER CARTRIDGE, filed September 30, 2015, published as U.S. Patent Application Publication No. 2017/0086842 on March 30, 2017.

In the present example, adhesive layer 3104 is provided on buttress body 3102 in order to adhere buttress body 3102 to underside 3065 of anvil 3060. Similarly, adhesive layer 3114 is provided on buttress body 3112 in order to adhere buttress body 3112 to deck 3073 of staple cartridge 3070. Adherence of the buttress body 3102 to underside 3065 of anvil 3060 or to deck 3073 of staple cartridge 3070 can occur through a variety of mechanisms including but not limited to a pressure sensitive adhesive. In some versions, each adhesive layer 3104, 3114 comprise a pressure sensitive adhesive material. Examples of various suitable materials that may be used to form adhesive layers 3104, 3114 are disclosed in U.S. Patent Application Serial No. 14/667,842, entitled METHOD OF APPLYING A BUTTRESS TO A SURGICAL STAPLER, filed March 25, 2015, published as U.S. Patent Application Publication No. 2016/0278774 on September 29, 2016. Alternatively, any other suitable materials may be used. It should be understood that the term "adhesive," as used herein, may include (but is not limited to) tacky materials and also materials that are pliable or wax-like and adhere to a complex geometry via deformation and conformance. Some suitable adhesives may provide such pliability to adhere to a complex geometry via deformation and conformance without necessarily providing a high initial tack. In some instances, adhesives with lower tackiness may be removed more cleanly from surfaces. Various suitable materials that may be used to form adhesive layers 3104, 3114 will be apparent to those of ordinary skill in the art in view of the teachings herein.

As noted above, buttress assembly 3100 may be applied to the underside 3065 of anvil 3060, and buttress 3110 may be applied to deck 3073 of staple cartridge 3070, before tissue is positioned in end effector 3040, and before end effector 3040 is actuated. Because end effector 3040 may be actuated many times during use of instrument 3010 in a single surgical procedure, it may be desirable to enable an operator to repeatedly and easily load buttress assemblies 3100 on underside 3065 of anvil 3060 during that single surgical procedure. In other words, because end effector 3040 may be actuated many times during use of instrument 3010 in a single surgical procedure, it may be insufficient to simply provide anvil 3060 pre-loaded with a buttress assembly 3100 without facilitating the re-loading of anvil 3060 with additional buttress assemblies 3100 after end effector 3040 has been actuated.

Similarly, those of ordinary skill in the art will recognize that staple cartridge 3070 will need to be replaced each time end effector 3040 is actuated. When end effector 3040 is actuated several times during use of instrument 3010 in a single surgical procedure, several staple cartridges 3070 may thus be used during that surgical procedure. It may seem that each of these staple cartridges 3070 may be provided with buttress assembly 3110 pre-loaded on deck 3073. However, there are some reasons why it may be undesirable to provide a staple cartridge 3070 with buttress assembly 3110 pre-loaded on deck 3073. In other words, it may be desirable to provide loading of buttress assembly 3110 on deck 3073 immediately prior to usage of staple cartridge in the surgical procedure, rather than loading buttress assembly 3110 on deck 3073 a substantial time prior to the surgical procedure. For instance, buttress assembly 3110 may not be compatible with the same sterilization techniques as staple cartridge 3070, such that it may present processing difficulties to package staple cartridge 3070 with buttress assembly 3110 pre-loaded on deck 3073. In addition, the material forming buttress assembly 3110 may have certain environmental sensitivities that staple cartridge 3070 does not have, such that it may be beneficial to enable buttress assembly 3110 and staple cartridge 3070 to be stored separately before use. Moreover, buttress assembly 3110 may not be warranted or otherwise desired in some surgical procedures, such that it may be desirable to enable a physician to easily choose whether staple cartridge 3070 should be loaded with buttress assembly 3110 before that staple cartridge 3070 is used in the surgical procedure.

In view of the foregoing, it may be desirable to enable an operator to repeatedly and easily load buttress assemblies 3100, 3110 on end effector 3040 on an ad hoc basis during a given surgical procedure. It may also be desirable to provide a device that provides support and protection to buttress assemblies 3100, 3110 before buttress assemblies 3100, 3110 are loaded on end effector 3040, in addition to that same device also enabling buttress assemblies 3100, 3110 to be easily loaded on end effector. The examples described below relate to various cartridge assemblies that provide such support, protection, and loading of buttress assemblies 3100, 3110. It should be understood that the following examples are merely illustrative. Numerous variations will be apparent to those of ordinary skill in the art in view of the teachings herein.

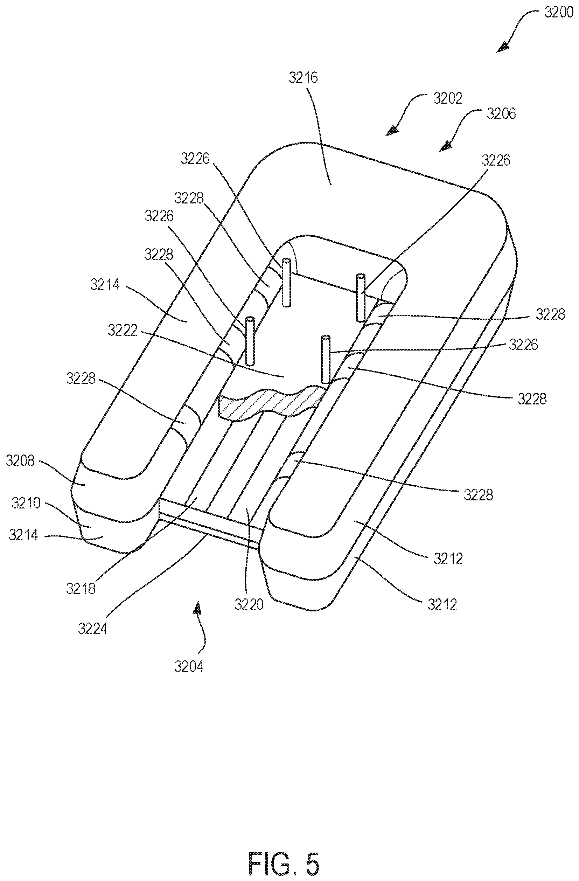

FIG. 5 illustrates a buttress applier cartridge 3200, according to at least one aspect of the present disclosure. The buttress applier cartridge 3200 can include a generally U-shaped housing assembly 3202 that defines an open end 3204 and a closed end 3206. In various embodiments, the housing assembly 3202 can include a top housing portion 3208 and a bottom housing portion 3210 that are coupleable together to form an outer shell of the housing assembly 3202. The top housing portion 3208 and the bottom housing portion 3210 each include a first leg 3212, a second leg 3214, and a connecting portion 3216 that connects the first leg 3212 to the second leg 3214 at the closed end 3206. The top housing portion 3208 and the bottom housing portion 3210 can be coupled with any suitable coupling mechanism, such as with snap-fit, latches, or press-fit, as examples. In one example embodiment, the housing assembly 3202 can include various internal components, such as those described in U.S. Patent No. 10,342,542.

The buttress applier cartridge 3200 can further include a support platform 3218 positioned between the first legs 3212 and second legs 3214 and that generally extends from the connecting portion 3216 of the housing assembly 3202 towards the open end 3204. In one aspect, the support platform 3218 can be manufactured out of any suitable, compressible material such that the support platform 3218 is compressible when force is applied thereto. In various other embodiments, the support platform 3218 can be rigid as opposed to compressible. In various embodiments, the support platform 3218 can be supported by the housing assembly 3202. In one example embodiment, the support platform 3218 can include a lip around the perimeter thereof that is captured between the top housing portion 3208 and the bottom housing portion 3210 when the top housing portion 3208 and bottom housing portion 3210 are coupled together. In other embodiments, the support platform 3218 can be coupled to the housing assembly 3202 in any suitable manner such that the support platform 3218 is substantially supported relative to the housing assembly 3202 when a force is applied thereto.

In various embodiments, the support platform 3218 can include a substantially planar top surface 3220 that can support a first buttress layer 3222 and a substantially planar bottom surface that can support a second buttress layer 3224. The first and second buttress layers 3222, 3224 can be removably coupled to the support platform 3218 by any suitable means, such as an adhesive, such that the first and second buttress layers 3222, 3224 are supported on their support platforms until the first and second buttress layers 3222, 3224 interface with an end effector of a surgical instrument, as will be described in more detail below.

In various aspects, the buttress applier cartridge 3200 can further include a plurality of suture legs 3226. In one example embodiment, as is shown in FIG. 5, the suture legs 3226 can extent from the first buttress layer 3222. The suture legs 3226 can be coupled to the first buttress layer 3222 in any suitable manner such that the suture legs 3226 can support the first buttress layer 3222 and, in various embodiments, such that movements of the suture legs 3226 causes movement of the first buttress layer 3222. In one example embodiment, two laterally offset suture legs 3226 form a continuous suture that is threaded through the first buttress layer 3222. In another example embodiment, two laterally offset suture legs 3226 form a continuous suture that supports a bottom surface of the first buttress layer 3222. Stated another way, the continuous suture extends underneath the first buttress layer and is positioned between the bottom surface of the first buttress layer 3222 and the top planar surface 3220 of the support platform 3218. In another example embodiment, each suture leg 3226 is coupled to the first buttress layer 3222 at discrete locations, such as by adhesive or embedded in the first buttress layer 3222, or any other suitable coupling mechanism.

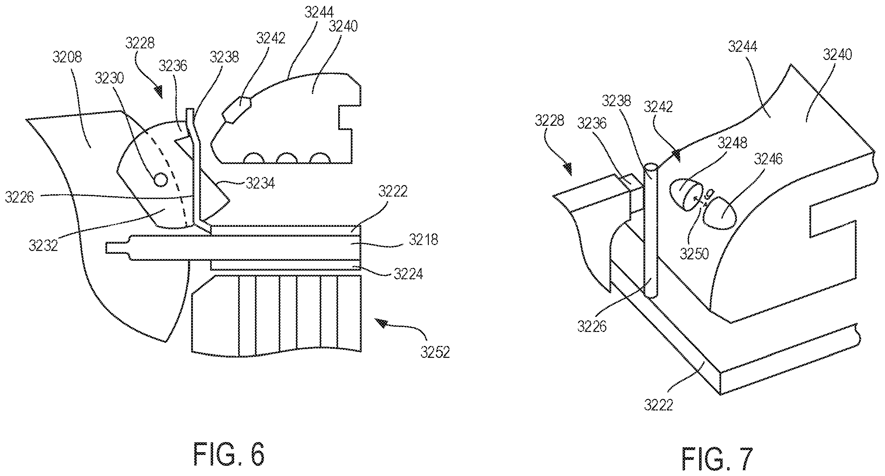

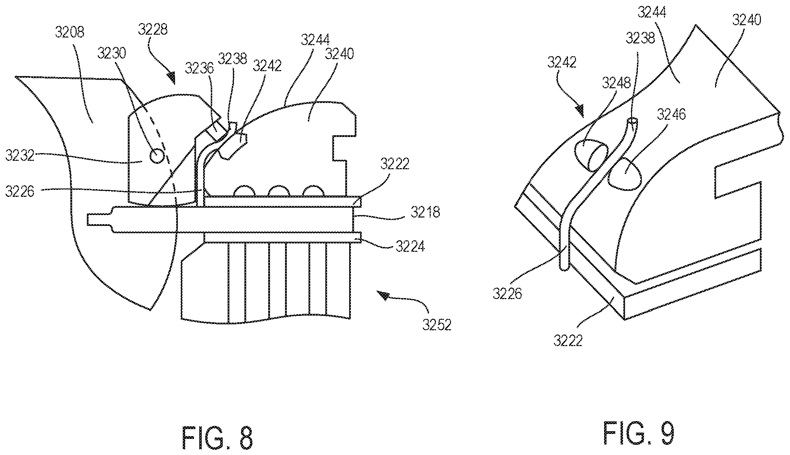

In one aspect, the buttress applier cartridge 3200 can further include a plurality of suture appliers 3228 (FIG. 5 shows the general position of the suture appliers 3228, while FIGS. 6-9 show an example embodiment of the structure of the suture appliers 3228). In various embodiments, each suture applier 3228 can be rotatably coupled to the buttress applier cartridge 3200. In one example embodiment, as seen in FIGS. 6 and 8, the suture appliers 3228 can be rotatable coupled to the top housing portion 3208 by pins 3230. The suture appliers 3228 can include a body portion 3232, a camming surface 3234, and an arm 3236 extending from the body portion 3232. Ends 3238 of each suture leg 3226 extending from the first buttress layer 3222 can removably couple to a corresponding arm 3236 of a suture applier 3228, such as with an adhesive, as an example.

As is shown in FIGS. 6-8, an anvil 3240 of an end effector can interface with the buttress applier cartridge 3200. The anvil 3240 can include a plurality of suture grabbers 3242 positioned on an outer, top surface 3244 thereof. As shown in FIGS. 7 and 9, the suture grabbers 3242 can include a first arm 3246 and a second arm 3248 spaced apart from the first arm 3246 such that a gap 'g' is defined therebetween. In one aspect, the gap 'g' is defined such that the ends 3238 of the suture legs 3226 can be received between the first arm 3246 and the second arm 3248 and would be press-fit and held by the suture grabber 3242. In various embodiments, the suture grabber 3242 can include an adhesive positioned between the first arm 3246 and the second arm 3248 on a receiving surface 3250 of the suture grabber 3242 such that, when an end 3238 of a suture leg 3226 is pressed between the first arm 3246 and the second arm 3248 (as is shown in FIG. 9), the end 3238 would at least be partially adhered to the anvil 3240, as well as being press-fit between the first arm 3246 and the second arm 3248, thus increasing the suture grabbers 3242 ability to hold the suture legs 3226.

In operation, as is shown in FIGS. 6-9, the anvil 3240 can be moved toward the first buttress layer 3222. Outer edges of anvil 3240 can contact and ride along camming surfaces 3234 of suture appliers 3228. In one example embodiment, the suture appliers 3228 are spaced along the buttress applier cartridge 3200 such that the suture appliers 3228 collectively cause the anvil 3240 to longitudinally align with the buttress applier cartridge 3200. In other embodiments, the buttress applier cartridge 3200 includes an alignment feature that allows the anvil 3240 to be positioned within the buttress applier cartridge 3200 such that each of the suture grabbers 3242 of the anvil 3240 is aligned with a corresponding suture applier 3228. In one example embodiment, the anvil 3240 is sized such that the anvil 3240 can abut against the connecting portion 3216 of the housing assembly 3202, causing the suture grabbers 3242 of the anvil 3240 to align with a corresponding suture applier 3228.

Continuing from above, outer edges of anvil 3240 can contact and ride along camming surfaces 3234 of suture appliers 3228. The force on the camming surfaces 3234 can cause the suture appliers 3228 to rotate about their pins 3230, causing the arms 3236, and thus, the ends 3238 of the suture legs 3226, to rotate towards the anvil 3240. Continued rotation of the suture applier 3228 can cause the suture appliers 3228 to force the ends 3238 of the suture legs 3226 into the gap 'g' between the first arms 3246 and the second arms 3248 of the suture grabbers 3242. As the anvil 3240 contacts first buttress layer 3222, the suture appliers 3228 can reach a completed rotated position, as is shown in FIG. 8 and the suture appliers 3228 completely force ends 3238 of suture legs 3226 into the suture grabbers 3242. Once the ends 3238 of the suture legs 3226 have been pressed into the suture grabbers 3242, the anvil 3240 can be moved away from the buttress applier cartridge 3200. In one example embodiment, the suture appliers 3228 can include a torsional spring such that, as the anvil 3240 is moved away from the support platform 3218, the arms 3236 of the suture appliers 3228 can be biased away from the anvil 3240 towards a non-rotated position, as is shown in FIG. 6. As the arms 3236 of the suture appliers 3228 rotate away from the anvil 3240, the suture grabbers 3242 can hold the ends 3238 of the suture legs 3226, causing the ends 3238 to release from arms 3236 of the suture appliers 3228. The suture legs 3226 and the suture grabbers 3242 collectively function to retain the first buttress layer 3222 against the anvil 3240.

Other than just the suture legs 3226 and the suture grabbers 3242, other suitable means for coupling the first buttress layer 3222 to the anvil 3240 can be used in combination with the suture legs 3226 and suture grabbers 3242. In one example embodiment, the first buttress layer 3222 can include an adhesive on a surface thereof such that, when the anvil 3240 is brought into contact with the first buttress layer 3222 (as is shown in FIG. 8), a tissue contacting surface of the anvil 3240 and the first buttress layer 3222 can be at least partially adhered together. Other means of coupling the anvil 3240 to the first buttress layer 3222 are described throughout the present application and can be used in connection with the buttress applier cartridge 3200.

While the figures and the above-provided description describe using suture appliers 3228 to couple a buttress layer 3222 to an anvil 3240, it should be understood that other embodiments are envisioned where the buttress applier cartridge 3200 can include suture appliers 3228 on the bottom surface on the buttress applier cartridge 3220 such that a buttress layer can be coupled to a staple cartridge positioned within an elongate channel of an end effector. In one example embodiment, similar to the anvil 3240, an elongate channel of the end effector can include suture grabbers positioned on an outside surface thereof. The bottom surface of the buttress applier cartridge 3200 can include suture appliers 3228 and suture legs 3226 that support the second buttress layer 3224. In one example embodiment, as the elongate channel and staple cartridge are brought toward the second buttress layer, the suture appliers 3228 on the bottom surface of the buttress applier cartridge 3200 can force suture legs 3226 into suture grabbers on the elongate channel, similar to what was described above in regards to the anvil 3240. In other example embodiments, as shown in FIGS. 6-8, the bottom surface of the buttress applier cartridge 3220 may not include suture appliers 3228; rather just a buttress layer 3224 that can interface with the staple cartridge 3252. Other example embodiments are envisioned where other suitable means can be included on the bottom surface of the buttress applier cartridge 3200 to assist in coupling the second buttress layer 3224 to the staple cartridge 3252.

As described above, the support platform 3218 can be manufactured out of a compressible material. In operation, while the anvil 3240 is brought towards the first buttress layer 3222, staple cartridge 3252 positioned in the elongate channel of the end effector can be brought towards the second buttress layer 3224 of the buttress applier cartridge 3200, as shown in FIGS. 6 and 8. The anvil 3240 and the staple cartridge 3252 collectively compress against buttress layers 3222, 3224 towards the support platform 3218, helping maintain the position of the buttress applier cartridge 3200 and providing additional support in adhering the buttress layers 3222, 3224 to anvil 3240 and staple cartridge 3252, respectively.

After the buttress layers 3222, 3224 have been applied to the anvil 3240 and staple cartridge 3252, respectively, in one example embodiment, new buttress layers can be positioned on the planar surfaces of the support platform 3218 and the buttress applier cartridge 3200 can be utilized again. In another example embodiment, the support platform 3218 can be removed and replaced with another support platform 3218 that already includes new buttress layers 3222, 3224 positioned thereon. Other example embodiments are envisioned where the buttress applier cartridge 3200 is disposable after a single use.

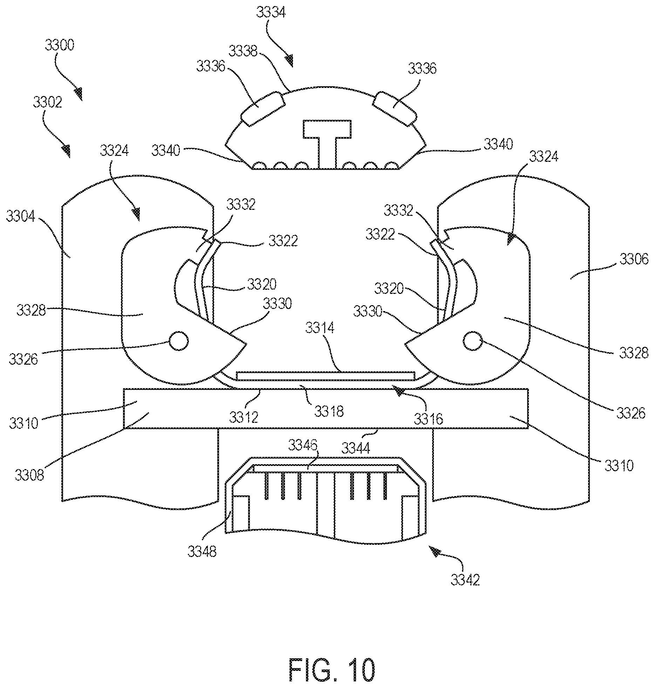

Referring now to FIG. 10, another buttress applier cartridge 3300 is provided, according to at least one aspect of the present disclosure. The buttress applier cartridge 3300 can include a housing assembly 3302 that can include a first leg 3304 and a second leg 3306. In one example embodiment, the housing assembly 3302 can be of unitary construction; however, other example embodiments are envisioned where the housing assembly 3302 is not of unitary construction. In one example embodiment, the housing assembly 3302 can include a top housing portion and a bottom housing portion that are coupleable together to form an outer shell of the housing assembly 3302, similar to housing assembly 3202. In various embodiments, the constructions of the buttress applier cartridge 3300 can be substantially similar to buttress applier cartridge 3200 apart from the difference referenced below.

The buttress applier cartridge 3300 can further include a support platform 3308 positioned between the first leg 3304 and second leg 3306. The support platform 3308 can be manufactured out of any suitable material such that the support platform 3308 is compressible when force is applied thereto. In various other embodiments, the support platform 3308 could be rigid as opposed to compressible. In various embodiments, the support platform 3308 can be supported by the housing assembly 3302. In one example embodiment, the support platform 3308 could include a lip 3310 around the perimeter thereof that is captured and held by the housing assembly 3302. In one embodiment where the housing assembly 3302 isn't of unitary construction, the lip 3310 can be positioned between a top housing portion and a bottom housing portion when the top housing portion and bottom housing portion are coupled together. In other embodiments, the support platform 3308 can be coupled to the housing assembly 3302 in any suitable manner such that the support platform 3308 is substantially supported relative to the housing assembly 3302 when a force is applied thereto.

The support platform 3308 can include a substantially planar top surface 3312 that can support a first buttress layer 3314. The first buttress layer 3314 can be removably coupled to the support platform 3218 by any suitable means, such as an adhesive, such that the first buttress layer 3314 is supported on their support platform 3308 until the first buttress layer 3314 interface with an end effector of a surgical instrument, as will be described in more detail below.

The buttress applier cartridge 3300 can further include a suture 3316 that includes a suture base 3318 and suture legs 3320 extending from the suture base 3318. In one example embodiment, as is shown in FIG. 10, the suture base 3318 can be positioned between the first buttress layer 3314 and the top surface 3312 of the support platform 3308 such that the suture 3316 supports the first buttress layer 3314. While one suture 3316 is shown and described, it should be understood that a plurality of sutures 3316 can be utilized to support the first buttress layer 3314.

The buttress applier cartridge 3300 can further include a plurality of suture appliers 3324. Each suture applier 3324 can be rotatably coupled to the buttress applier cartridge 3300. In one example embodiment, as seen in FIG. 10, the suture appliers 3324 can be rotatable coupled to the legs 3304, 3306 by pins 3326. The suture appliers 3324 can include a body portion 3328, a camming surface 3330, and an arm 3332. Ends 3322 of each suture leg 3320 can removable couple to a corresponding arm 3332 of a suture applier 3324, such as with an adhesive, as an example.

Similar to what was described for FIGS. 6-8, an anvil 3334 of an end effector of a surgical instrument can interface with the buttress applier cartridge 3300. The anvil 3334 can include a plurality of suture grabbers 3336 positioned on an outer, top surface 3338 thereof. In one example embodiment, the suture grabbers 3336 can be similar to suture grabbers 3242 described herein above. In various other embodiments, the suture grabbers 3242 can be similar to the suture grabbers described in more detail elsewhere in the present application.

In operation, the anvil 3334 is moved toward the first buttress layer 3314. Outer edges of anvil 3340 can contact and ride along camming surfaces 3330 of suture appliers 3324. In one example embodiment, the suture appliers 3324 are spaced along the buttress applier cartridge 3300 such that the suture appliers 3324 collectively cause the anvil 3334 to longitudinally align with the buttress applier cartridge 3300. The camming force on the camming surfaces 3330 causes the suture appliers 3324 to rotate about their pins 3326, causing the arms 3332, and thus, the ends 3322 of the suture legs 3320 to rotate towards the anvil 3334.

Continued rotation of the suture applier 3324 causes the suture appliers 3324 to force the ends 3322 of the suture legs 3320 into the suture grabbers 3336. As the anvil 3334 contacts first buttress layer 3314, the suture appliers 3324 can reach a complete rotated position and the suture appliers 3324 completely force ends 3322 of suture legs 3320 into the suture grabbers 3336. Once the ends 3322 of the suture legs 3320 have been pressed into the suture grabbers 3336, the anvil 3334 can be moved away from the buttress applier cartridge 3300. In one example embodiment, the suture appliers 3324 can include a torsional spring such that, as the anvil 3334 is moved away from the support surface 3308, the arms 3332 of the suture appliers 3324 can be biased away from the anvil 3334 towards a non-rotated position, as is shown in FIG. 10. As the arms 3332 of the suture appliers 3324 rotate away from the anvil 3334, the suture grabbers 3336 can hold the ends 3322 of the suture legs 3320, causing the ends 3322 to release from arms 3332 of the suture appliers 3324. As the anvil is moved away from the buttress appliers cartridge 3300, the base 3318 of the suture can support the bottom surface of the first buttress layer 3314, while the ends 3322 of the suture legs 3320 are held by the anvil, thereby retaining the first buttress layer 3314 against the tissue contacting surface of the anvil 3334.

As described above, the support platform 3308 can be manufactured out of a compressible material. In operation, while the anvil 3334 can be brought towards the first buttress layer 3314, a staple cartridge 3342 positioned in the elongate channel of the end effector can be brought towards the bottom surface 3344 of the support platform 3308. In one example embodiment, as is shown in FIG. 10, the staple cartridge 3342 can already be supplied with a buttress layer 3346 that is supported by a suture 3348. In other example embodiments, the bottom of the buttress applier cartridge 3300 can include suture appliers 3324 such that the staple cartridge 3342 can receive a buttress layer at the same time as the anvil 3334 receiving a buttress layer. In operation, as the anvil 3334 and the staple cartridge 3342 can collectively compress the support platform 3308, helping maintain the position of the buttress applier cartridge 3300 and providing additional support in adhering the buttress layer 3314 to anvil 3334.

As described above, the anvil and/or elongate channel of an end effector can be modified to include suture grabbers, such as suture grabbers 3242, 3336, that can receive and hold sutures in tension to hold a buttress against the anvil and/or elongate channel prior to firing the surgical instrument. As the surgical instrument is fired, a knife traveling within the end effector can cut through the buttress and the suture. When the surgical device is removed from the trocar, a free end of the suture can be removed from the suture grabber and another buttress can be applied to the surgical device using a buttress applier cartridge. In one example embodiment, as described above, the anvil can include a suture grabber 3242 that includes first arm 3246 and a second arm 3248 spaced from the first arm 3246 and that can releasably hold a suture therein.

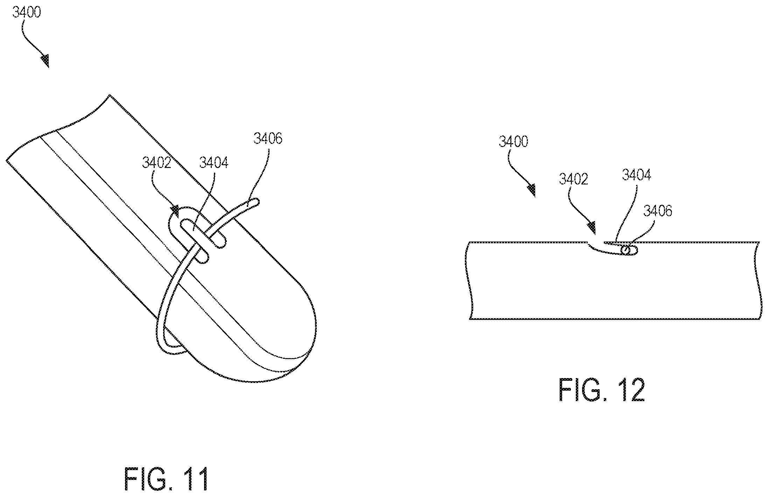

Another example embodiment of a suture grabber is shown in FIGS. 11 and 12, which illustrates an anvil 3400 that includes a cutout 3402 defined therein and a flap 3404 extending over the cutout 3402. The cutout 3402 and flap 3404 function in manner similar to that of a dental floss contain. In operation, a suture 3406 can be pulled through the cutout 3402 and wedged beneath the flap 3404 (shown most clearly in FIG. 12). The flap 3404 can be dimensioned such that the suture is retained within the cutout 3402, allowing the suture 3406 to be tensioned and held in place. The cutout 3402 and flap 3404 allows the suture 3406 to hold a buttress against the anvil 3400. In another embodiment, the cutout 3402 and flap 3404 can be included on an elongate channel of the end effector so as to allow a suture (or a plurality of sutures) to retain a buttress against a staple cartridge. While one cutout 3402 and flap 3404 is shown and described, it should be understood that the anvil (or elongate channel) can include a plurality of cutouts 3402 and flaps 3404 to allow a plurality of sutures to retain a buttress against the anvil (or elongate channel).

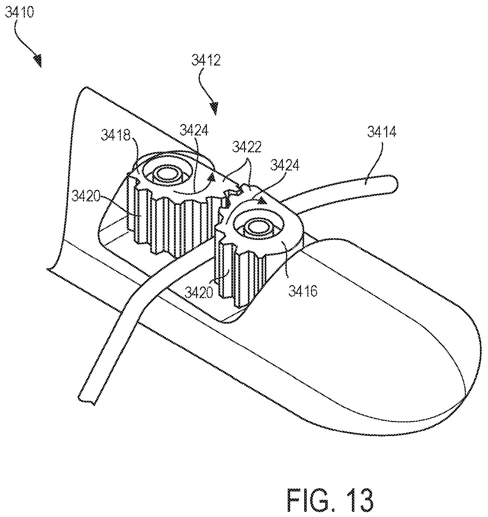

Another example embodiment of a suture grabber is shown in FIG. 13, which illustrates an anvil 3410 that includes a cam-cleat style lock 3412 that can hold a suture 3414 in tension. The cam-cleat lock 3412 can include a first cleat 3416 and a second cleat 3418, each of which includes a plurality of teeth 3420 and an arm 3422. The first cleat 3416 and second cleat 3418 can be rotatably coupled to the anvil 3410 and can be rotatable relative to each other between a captured configuration, where the arms 3422 of the first 3416 and second cleats 3418 contact each other (as is shown in FIG. 13), and an uncaptured configuration, where the arms 3422 of the first 3416 and second cleats 3418 are rotated away from each other. In the uncaptured configuration, a gap can be defined between the first 3416 and second cleats 3418 such that the suture 3414 can be threaded between the cleats 3416, 3418. Each of the cleats 3416, 3418 can further include a biasing mechanism, such as a torsional spring, such that each of the cleats 3416, 3418 can be biased towards the captured configuration.

In operation, each of the cleats 3416, 3418 can be moved toward the uncaptured configuration (as indicated by arrows 3424). A suture 3414 can be threaded between the cleats 3416, 3418 in the gap that is defined between the cleats 3416, 3418 when the cleats 3416, 3418 are in the uncaptured configuration. Once the suture 3414 has been pulled through the cleats and a sufficient amount of tension has been achieved in the suture 3414, the cleats 3416, 3418 can be released such that the cleats 3416, 3418 are biased towards the captured configuration. The arms 3422 of the cleats engage the suture 3414 (as is shown in FIG. 13) therebetween and maintain the suture 3414 in tension.

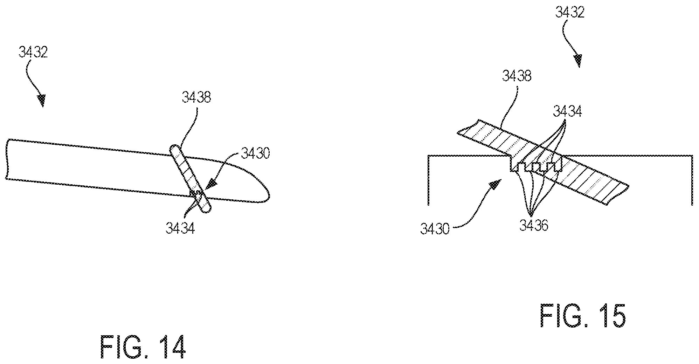

Another example embodiment of a suture grabber is shown in FIGS. 14 and 15, which illustrate a small cutout 3430 defined in an anvil 3432. The cutout 3430 can include a plurality of alternating teeth 3434 and grooves 3436 (shown most clearly in FIG. 15) such that, when a suture 3438 is tensioned and pulled through the cutout 3430, the strands of the suture 3438 are intermeshed and captured by the teeth 3434 and grooves 3436. The teeth 3434 and grooves 3436 can hold and restrict movement of the suture 3438 when the strands are captured, thus allowing the suture 3438 to maintain tension and hold a buttress against the anvil 3432.

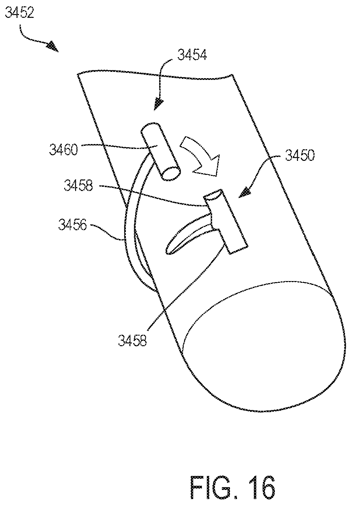

Another example embodiment of a suture grabber is shown in FIG. 16, which illustrates a shaped groove 3450 defined in an anvil 3452. The groove 3450 can be shaped to receive a correspondingly-shaped T-tag 3454 at an end of a suture leg 3456. In one example embodiment, the suture leg 3456 can be tensioned and stretched such that the T-tag 3454 is pulled over the groove 3450. Once sufficiently tensioned, the T-tag 3454 of the suture leg 3456 can be released such that the T-tag 3454 is dropped into and captured by the groove 3450. Walls 3458 of groove 3450 can contact and hold the transverse portion 3460 of the T-tag 3454 within the groove 3450, keeping the suture leg 3456 in tension and maintaining the T-tag 3454 in place during the surgical procedure. In another example embodiment, the T-tag 3454 and corresponding groove can have similar geometries with tight tolerances such that the T-tag 3454 can be press-fit into the groove 3450 to maintain tension in the suture leg 3456, but loose enough so that the T-tag 3454 can be released from the anvil 3452 after completion of the surgical stapling procedure. While a T-tag is shown and described, any suitable geometry and shape of tag and groove can be used at the end of the suture leg 3456, such as a square shape or star shape, as example.

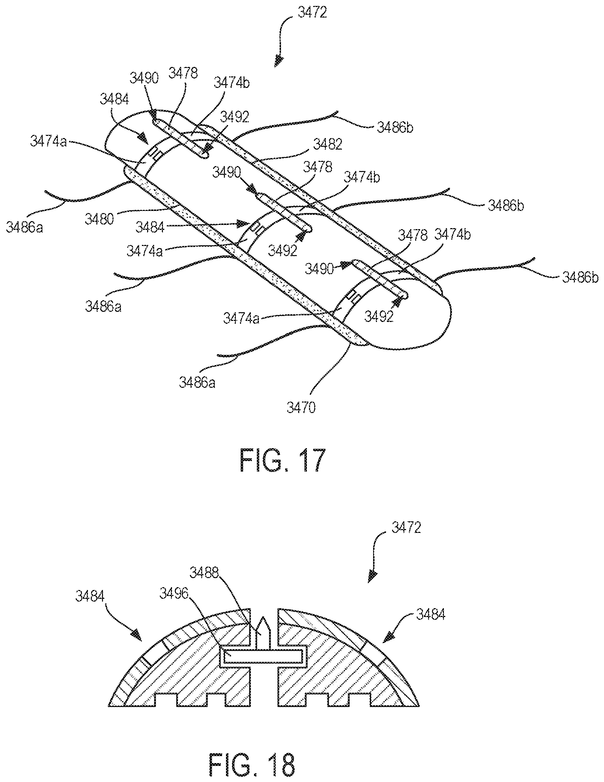

Another example embodiment for securing a buttress 3470 to an anvil 3472 is shown in FIG. 17. As shown in FIG. 17, the anvil 3472 can include a plurality of grooves 3474a, 3474b longitudinally spaced along an outside, top surface of the anvil. The first grooves 3474a and second grooves 3474b can extend towards a suture knife pocket 3478 from a first lateral side 3480 of the anvil 3472 and a second lateral side 3482 of the anvil 3472, respectively. Each of the grooves 3474a, 3474b can include suture pinch feature 3484, which can capture and hold suture legs, as will be explained in more detail below.

In various embodiments, the buttress 3470 can include a plurality of suture legs 3486a, 3486b extending therefrom. The suture legs 3486a, 3486b can be coupled to or support the buttress 3470 in any suitable manner such that the suture legs 3486a, 3486b are able to maintain the buttress 3470 against the anvil 3472. In various embodiments, each suture leg 3486a, 3486b can be positioned within an adjacent groove 3474a, 3474b and be held by the suture pinch feature 3484 within the grooves 3474a, 3474b. In one aspect, suture legs 3486a, 3486b in laterally offset grooves 3474a, 3474b can be tensioned and coupled together in any suitable manner, such as by tying the ends of the suture legs 3486a, 3486b together in a knot. Once tied, the coupled suture legs 3486a, 3486b form a continuous suture that extends from a first side of the buttress 3470, through a groove 3474a, the suture knife pocket 3478, and a groove 3474b to a second side of the buttress 3470.

As shown in FIG. 18, each suture knife pocket 3478 can include a suture knife 3488. Each suture knife 3488 is movable within and through the suture knife pocket 3478 between a proximal position 3490 and a distal position 3492. In one example embodiment, as the suture knife 3488 moves between the proximal position 3490 and the distal position 3492, the suture knife 3488 severs the coupled suture legs 3486a, 3486b that extend over the suture knife pocket 3478. In various embodiments, the suture knife 3488 can move from the proximal position 3490 toward the distal position 3492 based on movement of a firing member, such as firing beam 3082, within the end effector. In one example, embodiment the firing member can abut a base portion 3496 of the suture knife 3488 as the firing member moves within the end effector to cut and staple tissue positioned therein. In other embodiments, the suture knives 3488 can be positioned at the distal positions 3492 of the suture knife pockets 3478 such that, as the firing member is retracted after the cutting and stapling procedure, the firing member can abut the base portions 3496 and move the suture knives 3488 proximally, severing the coupled suture legs 3486a, 3486b. The above-described embodiments allow for the progressive release of the buttress 3470 from the anvil 3472.

As described above, the buttress applier cartridge 3200 may be utilized to apply buttress layers to an anvil and a deck of a staple cartridge before tissue is positioned in an end effector and before end effector is actuated. Because end effector may be actuated many times during use of instrument and multiple staple cartridges may be used, it may be desirable to enable an operator to repeatedly and easily load buttress assemblies onto an anvil, while simultaneously loading the elongate channel of the end effector with a new staple cartridge that includes a buttress layer. In other words, because end effector may be actuated many times during use of instrument in a single surgical procedure, it may be desirable to include a buttress applier cartridge that is a 'one stop shop' for both reloading the end effector with a new staple cartridge that already includes a buttress layer and applying a buttress layer to an anvil.

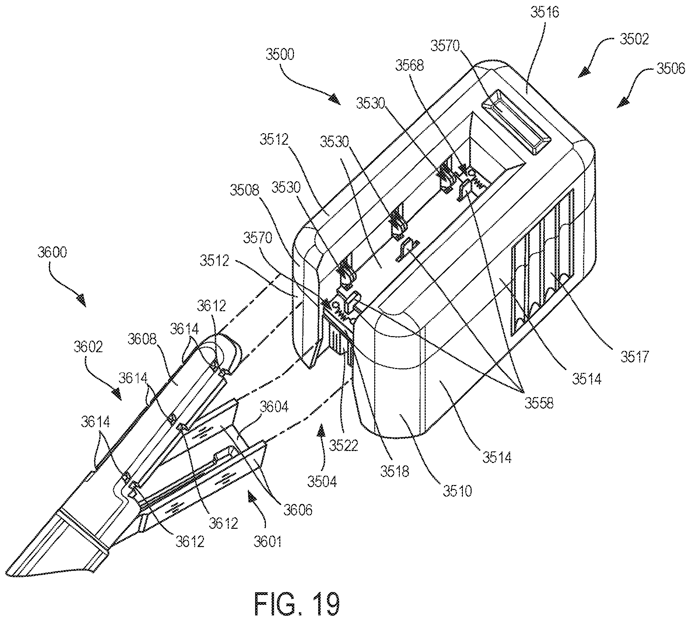

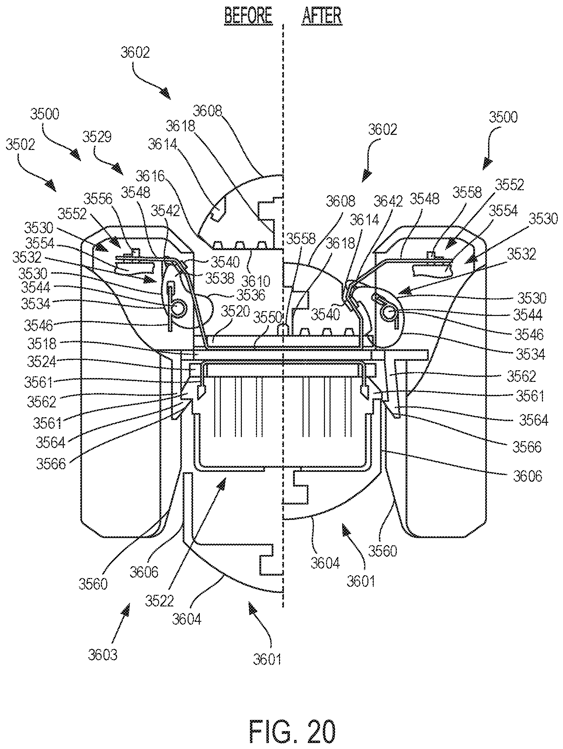

In one example embodiment, referring to FIG. 19, an end effector 3600 is provided that includes an elongate channel 3601 and an anvil 3602. The elongate channel 3601 includes a base 3604 and sidewalls 3606 extending upwardly from the base. The elongate channel 3601 is sized and configured to receive a staple cartridge therein that can be removably replaceable over the course of a surgical procedure. The anvil 3602 can include an outer surface 3608 and a tissue contacting surface 3610 (seen in FIG. 20). The outer surface 3608 of the anvil 3602 can include a plurality of notches 3612 and recessed pockets 3614, as will be discussed in more detail below. The end effector 3600 can interface with a buttress applier cartridge 3500, as will be discussed in more detail below, such that a buttress layer can be applied to the tissue contacting surface 3610 while a staple cartridge including a buttress layer is positioned within the elongate channel.

Continuing to refer to FIG. 19, a buttress applier cartridge 3500 accordingly to at least one aspect of the current disclosure is shown. The buttress applier cartridge 3500 can include a generally U-shaped housing assembly 3502 that defines an open end 3504 and a closed end 3506. The housing assembly 3502 includes a top housing portion 3508 and a bottom housing portion 3510 that are coupleable together to form an outer shell of the housing assembly 3502. The top housing portion 3508 and the bottom housing portion 3510 each include a first leg 3512, a second leg 3514, and a connecting portion 3516 that connects the first leg 3512 to the second leg 3514 at the closed end 3506. The top housing portion 3508 and the bottom housing portion 3510 can be coupled with any suitable coupling mechanism, such as with snap fit, latches, press-fit, as examples. In one example embodiment, the housing assembly 3502 can include various internal components, such as those described in U.S. Patent No. 10,342,542. In other example embodiments, the housing assembly 3502 can be of unitary construction as opposed to being separable into a top housing portion and a bottom housing portion. In various embodiments, the buttress applier cartridge 3500 can include grip features 3517 on each of the legs 3512, 3514 (grip feature only shown on second legs 3514) that allows a user to grip and position the buttress applier cartridge 3500.

The buttress applier cartridge 3500 can further include a support platform 3518 positioned between the first legs 3512 and second legs 3514 and that generally extends from the connecting portion 3516 of the housing assembly 3502 towards the open end 3504. The support platform 3518 can be manufactured out of any suitable material such that the support platform 3518 is compressible when force is applied thereto. In various other embodiments, the support platform 3518 could be rigid as opposed to compressible. In various embodiments, the support platform 3518 can be supported by the housing assembly 3502. In one example embodiment, the support platform 3518 could include a lip around the perimeter thereof that is captured between the top housing portion 3508 and the bottom housing portion 3510 when the top housing portion 3508 and bottom housing portion 3510 are coupled together. In other embodiments, the support platform 3518 can be coupled to the housing assembly 3502 in any suitable manner such that the support platform 3518 is substantially supported relative to the housing assembly 3502 when a force is applied thereto. In one example embodiment, the support platform 3518 can be integrally coupled to the housing assembly 3502. In various embodiments, the buttress applier cartridge 3500 can have similar construction attributes to the buttress applier cartridges described herein, such as buttress applier cartridges 3200, 3300.

The support platform 3218 can include a substantially planar top surface that can support a first buttress layer 3520 and a substantially planar bottom surface that interfaces with a buttress layer 3524 positioned on the deck on a staple cartridge 3522. In various embodiments, the first buttress layer 3520 and the second buttress layer 3524 can be removably coupled to the support platform 3218 by any suitable means, such as an adhesive, such that the first buttress layer 3520 and the second buttress layer 3524 are supported on the support platform 3518 until the first buttress layer 3520 and the staple cartridge 3522 interface with the end effector 3600 of a surgical instrument, as will be described in more detail below. In other example embodiments, only the first buttress layer 3520 is adhered to the support platform 3218, while the second buttress layer 3524 is merely supported by the staple cartridge 3522, such as by an adhesive or a suture. Other example embodiments of coupling the first buttress layer 3520 and the staple cartridge 3524 / second buttress layer 3524 to the buttress applier cartridge 3500 will be described below.

The buttress applier cartridge 3500 can further include a first loading region, or zone 3529 that can include a loading assembly for securing an absorbable layer to an anvil as the anvil approaches the absorbable layer. In various embodiments, the loading assembly can include a plurality of suture applying assemblies 3530 (shown most clearly in FIG. 20). Each suture applying assembly 3530 can include a suture applier 3532 that includes a suture applier body 3534, a camming surface 3536, an arm 3538, a plug 3540 extending from the arm 3538, and a knife 3542 extending from the arm 3538. Each suture applier body 3532 is rotatably coupled to one of the legs 3512, 3514 of the housing assembly 3502 about a pin 3544. The suture appliers 3532 are rotatable between a resting position (shown in the BEFORE side of FIG. 20) and an actuated position (shown in the AFTER side of FIG. 20). In various embodiments, a biasing mechanism, such as a torsion spring 3546, can be utilized to bias the suture appliers 3532 to the rested position. The suture appliers 3532 can be rotated toward the actuated position based on outer edges 3616 of the anvil 3602 riding along the camming surface 3536, as will be described in more detail below.

Each of the suture applying assemblies 3530 can further include a suture leg 3548. As is shown in FIG. 20, laterally offset suture applying assemblies 3530 each include a suture leg 3548 of one common, continuous suture 3550. The suture legs 3548 can be coupled to the plugs 3540 of the suture appliers 3532 such that movement of the plug 3540 causes movement in the suture legs 3548. In one aspect, the sutures 3550 can support the first buttress layer 3520 such that movement of the suture legs 3548 can move the first buttress layer 3520. In one example embodiment, as shown in FIG. 20, the suture 3550 extends from one suture applier assembly 3530, under a bottom surface of the first buttress layer 3520 and to a laterally offset suture applying assembly 3530.

Each of the suture applying assemblies 3530 can further include a suture anchor 3552. The suture anchors 3552 are fixably coupled to the housing assembly 3502 and include a base 3554 and an attachment portion 3556. In various embodiments, the suture legs 3548 can extend toward and couple to the attachment portions 3556 such that, as the suture leg 3548 are moved by the suture appliers 3532 toward the anvil 3602, as will be described in further detail below, the suture anchors hold the ends of the suture legs 3548, generating tension in the sutures 3550.

In addition, as reference above, the buttress applier cartridge 3500 can further include a second loading region, or zone 3603 that includes staple cartridge 3522 that can include a second buttress layer 3524. In various embodiments, the second buttress layer 3524 can be coupled to the staple cartridge 3522 such as by an adhesive or a suture 3561 applied to the staple cartridge 3522 prior to inserting the staple cartridge into the buttress applier cartridge 3500. In various other embodiments, the second loading region 3603 of the buttress applier cartridge can include suture appliers such that a staple cartridge can be loaded into the buttress applier cartridge and a buttress layer can be added to the staple cartridge therein.

In one aspect, the staple cartridge 3522 can include laterally extending fins 3561 that are held and supported by latches 3562 extending from the buttress applier cartridge 3500. The latches 3562 include arms 3564 that can hold the staple cartridge 3522 within the buttress applier cartridge 3500. The latches 3562 can further include camming surfaces 3566 that interface with the sidewalls 3606 of the elongate channel 3601 to release the staple cartridge 3522, as will be described in more detail below.

In operation, the anvil 3602 and the elongate channel 3601 of the end effector 3600 are brought toward the first loading region 3529 of the buttress applier cartridge 3500, as is shown in the BEFORE side of FIG. 20. The anvil 3602 can be moved toward the first buttress layer 3520 such that that outer edges 3616 of anvil 3602 can engage and ride along camming surfaces 3536 of the suture appliers 3532. As the outer edges 3616 of the anvil 3602 ride along the camming surfaces 3536, the anvil 3602 is brought into longitudinal alignment with the buttress applier cartridge 3500 due to the lateral spacing of the suture appliers 3632. Stated another way, the suture appliers 3532 are laterally positioned on the buttress applier cartridge 3500 such that the suture appliers 3532 collectively align the anvil 3602 with the support platform 3518 of the buttress applier cartridge 3500, ensuring that the anvil 3602 is properly aligned as the anvil 3602 approaches the first buttress layer 3520.

In one aspect, continued movement of the outer edges 3616 of the anvil 3602 along the camming surfaces 3536 causes suture appliers 3532 to rotate toward the actuated position, as described above. As the anvil 3602 is brought toward the first buttress layer 3520, the suture appliers 3532 can rotate and force the suture legs 3548 through the anvil notches 3612 and further forces the suture plugs 3540 into the recessed pockets 3614 defined in the anvil 3602. The suture plugs 3540 can be press-fit into the recessed pockets 3614 such that the suture plugs 3540, and thus, the suture legs 3548, are coupled to the anvil 3602. Further, as the suture plugs 3540 are forced into recessed pockets 3614, the suture anchors 3552 resist motion of the suture legs 3548 toward the recessed pockets 3614, causing tension to develop in the suture 3550, allowing the suture 3550 to securely press the first buttress layer 3520 against the tissue contacting surface 3610 of the anvil. As the suture appliers 3532 reach the actuated position, the knives 3542 on the suture appliers 3532 can contact and sever the suture legs 3548, releasing the sutures 3550 from the buttress applier cartridge 3500.

In addition to the above, in various embodiments, the buttress applier cartridge 3500 can further include anvil centering features 3558 that further assist in properly aligning the anvil 3602 with the first buttress layer 3520. The anvil centering features 3558 can extend from the support platform 3518 through the first buttress layer 3520 and can be received within the elongate channel 3618 of the anvil 3602. The anvil centering features 3558 are sized such that the anvil centering features 3558 force the anvil 3602 into proper alignment with the first buttress layer 3520.

At substantially the same time as the first buttress layer 3520 is being applied to the anvil 3602, the elongate channel 3601 of the end effector 3600 can be moved towards the second loading region 3603, as is shown in the BEFORE side of FIG. 20. The housing assembly 3502 of the buttress applier cartridge 3500 can include guide walls 3560 that are sized and positioned to abut the sidewalls 3606 of the elongate channel 3601 as the elongate channel 3601 is brought toward the staple cartridge 3522. The guide walls 3560 are sloped toward the base of the staple cartridge such that, if the sidewalls 3606 were to engage the guide walls 3560, the sidewalls 3606 would ride along the guide walls 3560 to become properly aligned with the camming surfaces 3566 of the latches 3562.

As the base 3604 of the elongate channel 3601 is brought toward the base of the staple cartridge 3522, the sidewalls 3606 can travel along the sidewalls of the staple cartridge 3522 and engage the camming surfaces 3566 of the latches 3562, as is shown in the AFTER side of FIG. 20. The sidewalls 3606 and latches 3562 are made of any suitable material of thickness such that, as the sidewalls 3606 engage the camming surfaces 3566, the arms 3564 of the latches 3562 can flex away from the fins 3561, releasing the staple cartridge 3522 from the buttress applier cartridge 3500. As the latches 3562 disengage the fins 3561, the base 3604 of the elongate channel 3601 can engage the base of the staple cartridge 3522 and the sidewalls 3606 engage the fins 3561 of the staple cartridge 3522, thus removably coupling the staple cartridge 3522 with the elongate channel 3601.

It should be understood that the anvil 3602 and elongate channel 3601 can be brought toward the buttress applier cartridge in the manner described above at substantially the same time such that, as the anvil 3602 engages the first buttress layer 3520 and the elongate channel 3601 engages the staple cartridge 3522, the anvil 3602 and the elongate channel 3601 can apply a sufficient force to the support platform 3518 such that a user ensures that enough force is generated to attach the suture legs 3548 to the anvil and removably seat the staple cartridge 3522 within the elongate channel 3601.

In addition, the buttress applier cartridge 3500 can further include a plurality of sensors that can sense or detector proper alignment of the end effector 3600 with the buttress applier cartridge 3500. In one example embodiment, the support platform 3518 can include a first sensor 3568 positioned near the closed end 3506 of the buttress applier cartridge 3500 and a second sensor 3570 positioned near the open end 3504 of the buttress applier cartridge 3500. As the tissue contacting surface 3610 of the anvil 3602 is brought into contact with the first buttress layer 3520, the first and second sensors 3568, 3570 can detect the alignment of the anvil 3602 relative to the support platform 3518 to determine if the anvil 3602 is properly aligned.

In one example embodiment, the first and second sensors 3568, 3570 can comprise resistors that form a circuit when the anvil 3602 is brought into properly alignment with the buttress applier cartridge. In another example embodiment, the first and second sensors 3568, 3570 can comprise Hall-effect sensors that detect magnets coupled to the anvil 3602. In various other embodiments, the first and second sensors 3568, 3570 can sense a position of the anvil 3602 prior to the tissue contacting surface 3610 reaching the first buttress layer 3520 or prior to the outer edges 3616 of the anvil 3602 engaging the camming surfaces 3536 of the suture appliers 3532, thus allowing a user to know if the anvil 3602 is properly longitudinally and laterally aligned within the buttress applier cartridge 3500 prior to moving the anvil 3602 toward the first buttress layer, ensuring that the suture appliers 3532 are not inadvertently actuated before the anvil 3602 is properly aligned. While the sensors described above were discussed regarding proper alignment of the anvil, various other embodiments are contemplated where sensors are utilized to ensure proper lateral and longitudinal alignment of the elongate channel 3601 within the buttress applier cartridge 3500.

In various embodiments, the buttress applier cartridge 3500 can further include a display 3572 in electrical communicate with the first and second sensors 3568, 3570. The display 3572 can provide a user with audible or visual feedback regarding information sensed by the first and second sensors 3568, 3570, such as whether or not the anvil 3602 and/or the elongate channel 3601 is properly aligned within the buttress applier cartridge. Various other embodiments are envisioned where the display 3572 can also provide additional information to the user regarding the buttress applier cartridge 3500, such as the size of the cartridge 3522 positioned therein, status information, or error messages if the buttress applier cartridge 3500 has damaged, or the like.

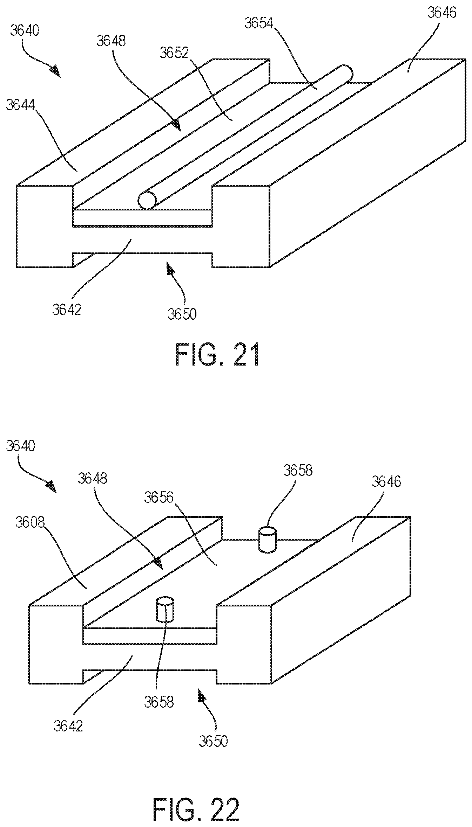

Referring now to FIGS. 21 and 22, another buttress applier cartridge 3640 is provided, in accordance with at least one aspect of the present disclosure. The buttress applier cartridge 3640 includes a support platform 3642, a first sidewall 3644, and a second sidewall 3646. The sidewalls 3644, 3646 extend in directions away from the support platform 3642 so as to define recessed areas 3648, 3650 on a top and bottom side of the buttress applier cartridge 3640.

Referring to FIG. 21, a first embodiment is provided where the first recessed area 3648 can include a buttress layer 3652 positioned on the planar support platform 3642. The buttress layer 3652 can include an elongate support 3654 extending from a surface of the buttress layer 3652. The elongate support 3654 is sized and manufactured such that the elongate support 3654 can be received within an elongate channel of an anvil, such as elongate channel 3618, as an example. As the anvil is brought toward buttress layer 3652, the elongate support 3654 can deform and be press-fit into the elongate channel so as to releasably couple the buttress layer 3652 to the anvil.

Referring to FIG. 22, a second embodiment is provided where the first recessed area 3648 can include a buttress layer 3656 positioned on the planar support platform 3642. The buttress layer 3656 can include a plurality of pins 3658 extending from a surface of the buttress layer 3652. The pins 3658 is sized and manufactured such that the pins 3658 can be received within the elongate channel of an anvil, such as elongate channel 3618, as an example. As the anvil is brought toward buttress layer 3656, the pins 3658 can deform and be press-fit into the elongate channel so as to releasably couple the buttress layer 3656 to the anvil. In various other embodiments, the pins can be laterally aligned as opposed to longitudinally aligned. In such embodiments, an anvil can include apertures defined in the tissue contacting surface such that the pins could be press-fit in the apertures. In this way, the buttress applier cartridge can be coupled to the anvil by way of pins, but the pins are positioned away from the elongate channel.

While the above-provided buttress applier cartridges were shown and described as having buttress layers positioned within the first recessed areas 3648, it should be understood that buttress layers can also be positioned against the support platform 3642 in the second recess area 3650. It should also be understood that the buttress layers 3652, 3656 can be utilized in a variety of buttress applier cartridges, such as buttress applier cartridges 3200, 3500 and any other buttress applier cartridges described herein.

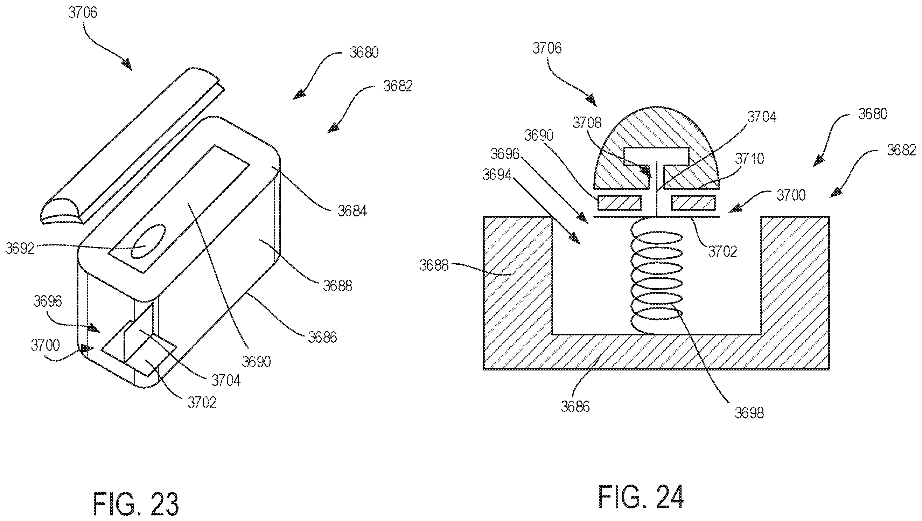

Referring now to FIG. 23, another buttress applier cartridge 3680 for attaching a buttress layer to an anvil is provided, in accordance with at least one aspect of the present disclosure. The buttress applier cartridge 3680 can include a housing assembly 3682 that includes a support platform 3684, a base 3686, and walls 3688 connecting the support platform 3684 to the base 3686. In various embodiments, the support platform 3684 is sized to support a buttress layer 3690 thereon. In various embodiments, both the support platform 3684 and the buttress layer 3690 can include a slot 3692 defined therein such that, when the buttress layer 3690 is properly aligned on the support platform 3684, the slots 3692 can align such that an opening is defined through the support platform 3684 into the interior 3694 of the housing assembly 3682. In various other embodiments, only the support platform 3684 may include a slot 3692, as will be described in more detail below.

Referring now to FIGS. 23 and 24, the housing assembly 3682 can further include a spring-loaded key assembly 3696 position within the interior 3694 of the housing assembly 3682. The spring-loaded key assembly 3696 can include a spring 3698 and a key 3700 coupled to the spring 3698. In various embodiment, the key 3700 can include a base 3702 and an alignment feature 3704 extending from the base 3702. The spring-loaded key assembly 3696 can be movable between a rested position (shown in FIG. 23), wherein the spring 3698 is compressed and the key 3700 is positioned within the housing assembly 3682, and an actuated position (shown in FIG. 24), where the spring is expanded and the key 3700 extends through the slots 3692 of the support platform 3684 and the buttress layer 3690. In embodiments where only the support platform 3684 includes a slot 3692, as referenced above, the alignment feature 3704 can include a blade such that, as the alignment feature 3704 moves toward the actuated position, the knife can move through the slot 3692 of the support platform 3684 and pierce through the buttress layer 3690.

As referenced above, the buttress applier cartridge 3680 can attach a buttress layer, such as buttress layer 3690, to an anvil 3706. In various embodiments, the anvil 3706 can include an elongate channel 3708 defined therein that can receive the alignment feature 3704 of the key 3700. In operation, the tissue contacting surface 3710 of the anvil 3706 can be pressed down onto the buttress layer 3690 positioned on the support platform 3684. Based on the pressure applied to the buttress layer 3690 and the buttress applier cartridge 3680, the spring-loaded key assembly 3696 can be actuated such that the spring-loaded key assembly 3696 moves from the resting position to the actuated position, as described above. In various embodiments, the support platform 3684 can include a pressure sensor that can sense the pressure the anvil 3706 applies to the housing assembly 3682. Once a threshold pressure is reached or exceeded by the anvil 3706, the spring-loaded key assembly 3696 can be actuated and moved to the actuated position. Various other embodiments are envisioned that can actuate the spring-loaded key assembly 3696 when sufficient force is provided by the anvil 3706.

In one aspect, when the spring-loaded key assembly 3696 is actuated, the alignment feature 3704 can extend from the housing assembly 3682 via the slots 3692 and into the elongate channel 3708 of the anvil 3706. The alignment feature 3704 can ensure that the buttress layer 3690 cannot be misaligned from its proper position on the tissue contacting surface 3710 of the anvil 3706 during the process of attaching the buttress layer 3690 to the anvil 3706. In various aspects, with the location of the slot 3692 in the buttress layer 3690 and the alignment feature 3704 within the buttress applier cartridge 3680, it can be ensured that misalignment of the buttress layer 3690 in translation along the major axis of the anvil 3706, in translation along the minor axis of the anvil 3706, or in rotation about the vertical axis through the anvil 3706 can be maintained. In various other embodiments, the slot 3692 of the buttress applier cartridge 3680 can be sized to be larger than the slot 3692 of the buttress layer 3690 such that the base 3702 of the key 3700 can extend from the housing assembly 3682 and abut the bottom surface of the buttress layer 3690, forcing the buttress layer 3690 against the tissue contacting surface 3710 of the anvil 3706, helping ensure the buttress layer 3690 doesn't move relative to the anvil 3706 during the alignment process.

Once the buttress layer 3690 has been properly aligned and affixed to the tissue contacting surface 3710 of the anvil 3706, such as with an adhesive, as an example, the anvil 3706 can be moved away from the buttress applier cartridge 3680, which can cause the spring-loaded key assembly 3696 to retract back to the resting position. In one example embodiment, the pressure sensor can continuously sense the pressure the anvil 3706 applies to the buttress applier cartridge 3680. When the applied pressure drops before a threshold level, such as the threshold level that activated the spring-loaded key assembly 3696, described above, a mechanism can retract the spring-loaded key assembly 3696 back to the resting position. In various embodiment, the threshold level to retract the spring-loaded key assembly 3696 can be less than the original threshold level that moved the spring-loaded key assembly 3696 to the actuated position, such that the level of pressure required to actuate the spring-loaded key assembly 3696 does not need to be maintained during the alignment process of the buttress layer 3690. In various embodiments, the threshold level to retract the spring-loaded key assembly 3696 could be the pressure sensor sensing zero force, thus indicating the anvil 3706 has been completed moved away from the buttress applier cartridge 3680.

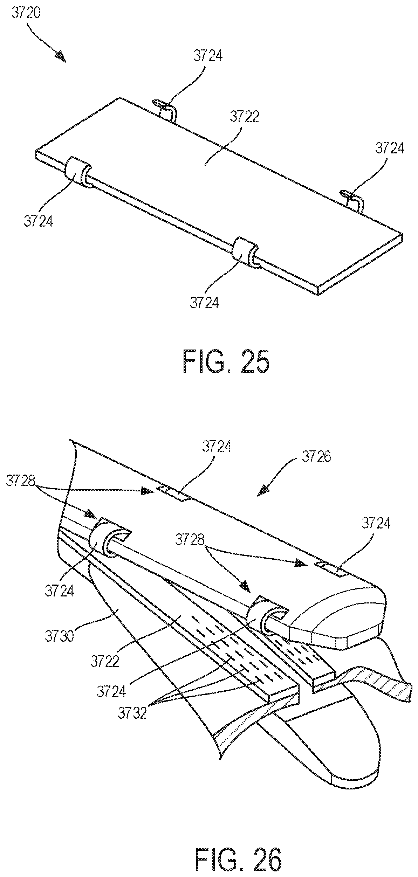

Referring now to FIG. 25, a buttress assembly 3720 is provided in accordance with at least one aspect of the present disclosure. The buttress assembly 3720 includes a buttress layer 3722 and a plurality of brackets 3724 extending from the buttress layer 3722. The brackets 3724 can be coupled to the buttress layer 3722 in an suitable manner, such as with an adhesive, such that, when a threshold force is applied to the brackets 3724, the brackets 3724 snap off of and release the buttress layer 3722, as will be described in more detail.

In various embodiments, referring to FIG. 26, the buttress assembly 3720 can be coupled to an anvil 3726 that includes a plurality of notches 3728 defined around a perimeter thereof. In one example embodiment, each bracket 3724 of the buttress assembly 3720 can align with a notch 3728 in the anvil 3726 such that, when each of the brackets 3724 are snapped into and captured by a corresponding notch 3728, the buttress layer 3722 can be brought into proper lateral and longitudinal alignment with the anvil 3726.

In one aspect, once the buttress assembly 3720 is coupled to the anvil 3726, by way of brackets 3724 and notches 3728, the anvil 3726 can be utilized in a surgical stapling procedure. After completion of a cutting and firing stroke, the buttress layer 3722 can be severed and stapled to tissue 3730, as is shown in FIG. 26. With the buttress layer 3722 held to the tissue 3730 by way of staples 3732 (three pointed to in FIG. 26), the anvil 3726 can be pulled away from the tissue 3730. In various embodiments, as the anvil 3726 is moved away from the tissue 3730, the brackets 3724 can be sufficiently stiff such that the brackets 3724 are held in notches 3728, causing the brackets 3724 to release from the buttress layer 3722. Once the brackets 3724 have been separated from the buttress layer 3722, a clinician can remove the brackets 3724 from the notches 3728 and reload the anvil 3726 with a new buttress assembly 3720. While brackets were shown and described that can couple the buttress layer 3722 to the anvil 3726, various embodiments are envisioned where the brackets 3724 are replaced or utilized in connection with other structures to couple the buttress layer 3722 to the anvil 3726. In various embodiments, these other structures could comprise pins, magnets, or slot mechanisms.

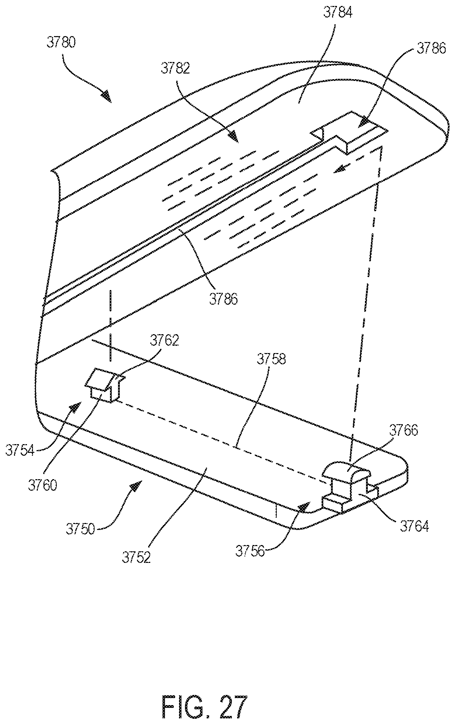

Referring now to FIG. 27, a buttress assembly 3750 is provided in accordance with at least one aspect of the present disclosure. The buttress assembly 3750 can include a buttress layer 3752, a coupling member 3754 and a bracket 3756. In one aspect, the coupling member 3754 and the bracket 3756 can be longitudinally aligned and laterally spaced along a central buttress axis 3758 such that, when the buttress layer 3752 is coupled to an anvil 3780, as will be described in more detail below, the buttress layer 3752 can cover the plurality of staple forming pockets 3782 on the tissue contacting surface 3784 of the anvil 3780.

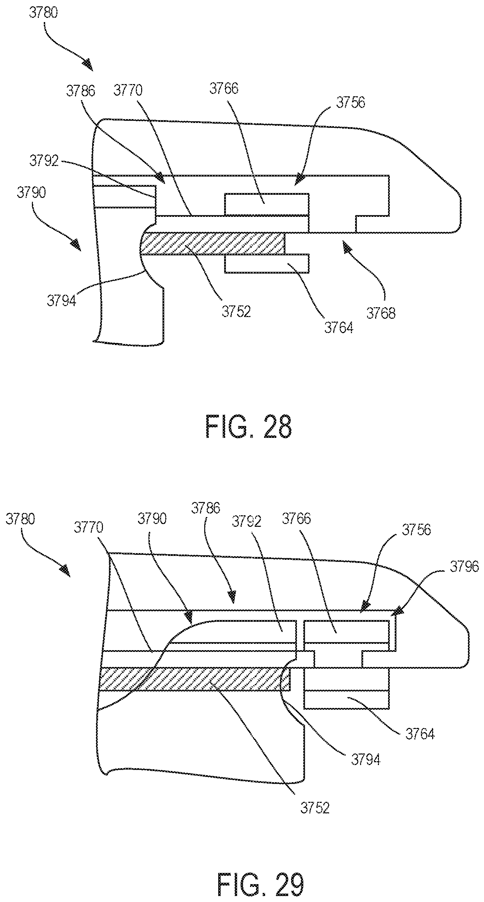

In various embodiments, the coupling member 3754 can include a base 3760 extending from the buttress layer 3752 and a head 3762 extending from the base 3760. The base 3760 can be coupled to the buttress layer 3752 in any suitable manner, such as with an adhesive. In other embodiments, the coupling member 3754 can of unitary construction with the buttress layer 3752 and comprise the same material as the buttress layer 3752. In various embodiment, the head 3762 can include any suitable shape such that the head 3762 can be press-fit into an elongate channel 3786 of the anvil 3780 and thereby retain the buttress layer 3752 to the anvil 3780. In one example embodiment, as is shown in FIG. 27, the head 3762 can include a triangular shape such that the coupling member 3754 forms an arrow-like shape. The triangular head 3762 can be pressed into the elongate channel 3786 such that the head 3762 can abut an inner contact surface 3770 of the anvil 3780 (shown in FIG. 28) and hold the coupling member 3754, and therefore, the buttress layer 3752, against the anvil 3780. Various other shapes are contemplated by the present disclosure that can be used as opposed to a triangular shaped head, such as a rectangular shaped head such that the coupling member 3754 forms a 'T' shape. While one coupling member 3754 is shown and described, a plurality of coupling members 3754 can be utilized to support the buttress layer 3752 and retain the buttress layer 3752 against the tissue contacting surface 3784.

In various embodiments, the bracket 3756 can include a base 3764 releasably coupled the buttress layer 3752 and a head 3766 extending from the base 3760. The base 3764 can be releasably coupled to the buttress layer 3752 in any suitable fashion, such as with an adhesive, such that when a threshold force is applied to the bracket 3756, the base 3764 can be released from the buttress layer 3752, as will be described in more detail below. In various embodiments, the bracket 3756 can comprise a material that is different than the buttress layer 3752. In one example embodiment, the bracket 3756 can be comprised of plastic. Other embodiments are envisioned where the bracket 3756 and the buttress layer 3752 comprise the same material.

In one aspect, the head 3766 can be received with an aperture 3768 at a distal end of the elongate channel 3786 of the anvil 3780 (illustrated by the dashed line in FIG. 27). Once inserted into the aperture 3768, the buttress assembly 3750 can be moved proximally (away from the tip of the anvil 3780) such that the head 3766 is positioned within the elongate channel 3786 of the anvil 3780 (as is shown in FIG. 28). The head 3766 can be sized such that the head 3766 abuts the contacting surface 3770 of the elongate channel 3786, thereby coupling the bracket 3756, and thus, the buttress layer 3752, to the anvil 3780. In various example embodiments, as the head 3766 is being received within the aperture 3768, the head 3762 of the coupling member 3754 can also being inserted into the elongate channel 3786, as described above. Once the head 3762 of the coupling member 3754 has been inserted into the elongate channel 3786 and the head 3766 of the bracket 3756 has been positioned within the aperture 3768, the buttress assembly 3750 can be pulled proximally such that the head 3762 of the coupling member 3754 and the head 3766 of the bracket 3756 are engaging the contact surface 3770 of the elongate channel 3786, thereby retaining the buttress layer 3752 to the anvil 3780.

Once the buttress layer 3752 is coupled to the anvil 3780, by way of the coupling member 3754 and the bracket 3756), the anvil 3780 can be used in a surgical stapling procedure, as described elsewhere herein. In one aspect, as shown in FIG. 27, a knife member 3790 can traverse the anvil toward the distal tip of the anvil 3780 during the surgical stapling procedure. In one example embodiment, the knife member 3790 can be similar to knife member 3080. The knife member 3790 can include an abutment surface that can engage the contact surface 3770 of anvil 3780 as the knife member 3790 traverses the elongate channel 3786. The knife member 3790 can also include a tissue cutting blade 3794 for cutting tissue and the buttress layer 3752 as the knife member traverses the elongate channel 3786.

In operation, the knife member 3790 can traverse distally through the elongate channel 3786 of the anvil 3780, severing the buttress layer 3752 and tissue positioned against the buttress layer 3752 with the blade 3794. When the blade 3794 encounters the coupling member 3754, the blade 3794 can severe the coupling member 3754, releasing the portion of the buttress layer 3752 to which the coupling member 3754 was coupled. In other example embodiments, the knife member 3790 abuts the coupling member 3754 such that the head 3762 of the coupling member 3754 is forced out of the elongate channel 3786 and is also severed by the blade 3794. In one aspect, the knife member 3790 is designed such that little to no remnants of the coupling member 3754 remain within the elongate channel 3786 after the knife member 3790 releases the coupling member 3754 from the anvil 3780.

Continuing from above, the knife member 3790 can continue to traverse distally through the elongate channel 3786 of the anvil 3780 and approach the bracket 3756, as is shown in FIG. 28. In various embodiments, the abutment surface 3792 can abut the head 3766 of the bracket 3756 and apply a sufficient force so as to release the bracket 3756 from the buttress layer 3752 (shown in FIG. 29). The abutment surface 3792 can force the bracket 3756 distally and force the head 3766 into a receiving area 3796 of the anvil 3780. After the knife member 3790 has positioned the bracket within the receiving area 3796, the knife member 3790 can be retracted proximally, leaving the bracket 3756 positioned within the receiving area 3796. At this point, a user of the surgical instrument can remove the anvil from the surgical site, manually remove the bracket 3756 from the receiving area 3796 of the anvil 3780 through the aperture 3768, and attached a new buttress assembly 3750 to the anvil 3780.

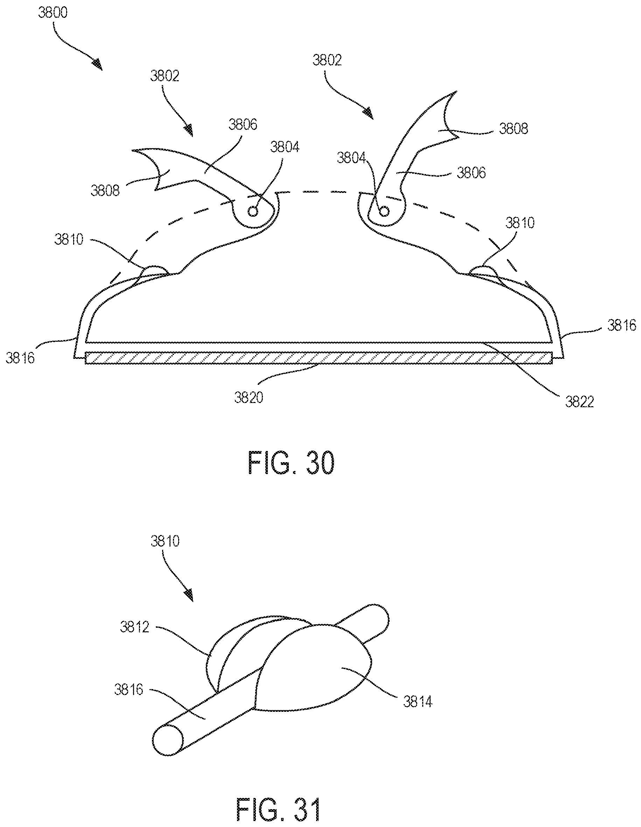

Referring now to FIG. 30, an anvil 3800 is provided in accordance with at least one aspect of the present disclosure. The anvil 3800 can include a plurality of levers 3802 rotatably coupled to the anvil 3800 about pins 3804. In various embodiments, the levers 3802 are friction clamps, meaning that the levers 3802 can retain their position about the pins 3804 until a sufficient force is applied to the levers 3802 to rotate them about the pins 3804. In one aspect, the levers 3802 can include a body 3806 and an arm 3808 extending from the body 3806.

In various embodiments, the anvil 3800 can further include a plurality of suture receivers 3810 (shown in more detail in FIG. 31). In various embodiments, the suture receives 3810 can include a first arm 3812 and a second arm 3814 spaced from the first arm 3812 to define a gap therebetween. In use, the suture receives 3810 can receive a suture leg 3816 between the first arm 3812 and the second arm 3814, as will be described in more detail below. In various embodiments, the first arm 3812 and the second arm 3814 are spaced apart such that the suture leg 3816 can be press fit and held between the first arm 3812 and the second arm 3814.

In one aspect, a buttress layer 3820 can interface with a tissue contacting surface 3822 of the anvil 3800, as shown in FIG. 30. In various embodiments, the buttress layer 3820 can include a plurality of suture legs 3816 extending therefrom. The suture legs 3816 can be coupled to the buttress layer 3820 in any suitable manner, such as manners described elsewhere herein, such that the suture legs 3816 can support the buttress layer 3820 against the tissue contacting surface 3822 of the anvil 3800.

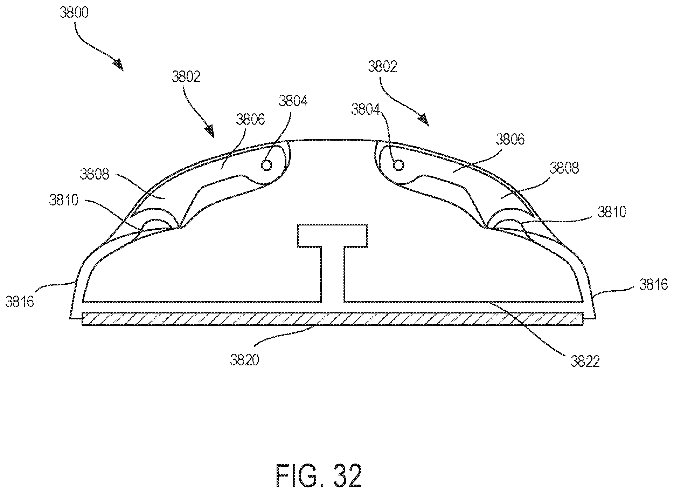

As shown in FIG. 30 and described above, the buttress layer 3820 can interface with the tissue contacting surface 3822 of the anvil 3800. In one aspect, as the buttress layer 3820 is interfacing with the tissue contacting surface, the suture legs 3816 can wrap about an outer surface of the anvil 3800 and extend toward the suture receivers 3810. In one example embodiment, the suture legs 3816 can be press fit within first and second arms 3812, 3814 of a corresponding suture receiver 3810. Once the suture legs 3816 are positioned within a suture receiver 3810, the levers 3802 can be rotated above pins 3804 such that arms 3808 of the levers 3802 engage the suture legs 3816, as is shown in FIG. 32, thereby releasably retaining the suture legs 3816, and therefore, the buttress layer 3820, to the anvil 3800. Once the buttress layer 3820 has be coupled to the anvil 3800, the anvil 3800 can be utilized in a surgical procedure as described elsewhere herein.

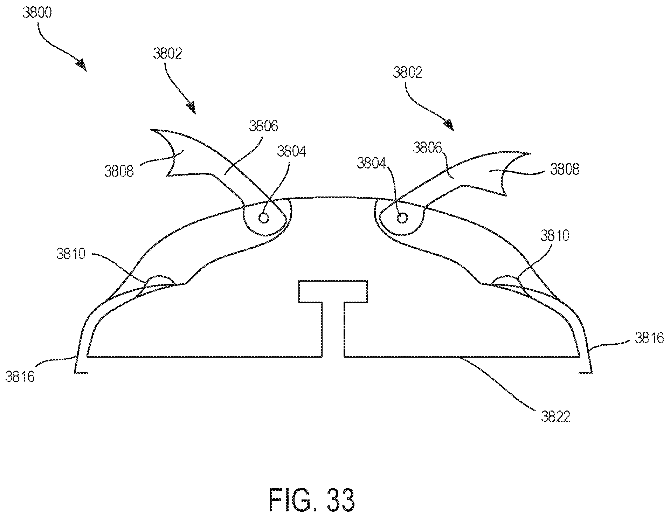

In one example embodiment, after the surgical instrument to which the anvil 3800 is being utilized with has been utilized in a stapling operation, the suture legs 3816 can be cut from the buttress layer 3820 in any suitable manner, such as with surgical scissors, as an example, and the anvil 3800 can be removed from the patient. In one aspect, as shown in FIG. 33, once the anvil 3800 has been removed from the patient, the arms 3808 of the levers 3802 can be rotated away from the anvil 3800 and the suture legs 3816 can be removed from the suture receivers 3810. After the suture legs have been removed from the suture receivers 3810, a new buttress layer 3820 including suture legs 3816 can be coupled to the anvil 3800 in the same manner as described above.

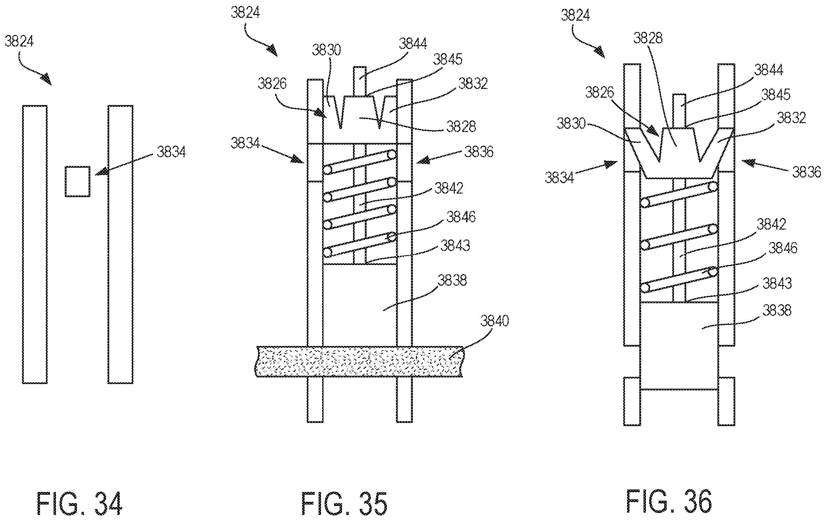

Referring now to FIGS. 34 through 36, a lockout mechanism 3824 is provided in accordance with at least one aspect of the present disclosure. In various embodiments, the lockout mechanism 3824 can be positioned within an anvil of an end effector. In various embodiments, the lockout mechanism 3824 can be positioned within the elongate channel and/or a staple cartridge of the end effector. In various embodiments, as will be discussed in more detail below, the lockout mechanism 3824 can interface with the elongate slot of the end effector such that the lockout mechanism can engage and prevent progress of a firing member through the elongate channel of the end effector. In one aspect, the lockout mechanism 3824 can interface with the I-beam slot of the end effector.

In various embodiments, the lockout mechanism 3824 can include a leaf spring 3826. In one aspect, the leaf spring 3826 can comprise a single, unitary structure. In other aspect, the leaf spring 3826 can comprise a grouping of like-structures grouped together to form the leaf spring 3826. In various embodiments, the leaf spring 3826 can be transitionable between a contracted configuration (shown in FIG. 35) and an expanded configuration (as shown in FIG. 36). While one leaf spring 3826 is shown and described, various other embodiments are envisioned where more than one leaf spring 3826 is utilized. In one aspect, the leaf spring 3826 can include a central body 3828, a first arm 3830 extending from the central body 3828 and a second arm 3832 extending from the central body 3828. The first and second arms 3830, 3832 can be rotatable relative to the central body 3828 to transition the leaf spring 3826 between the contracted configuration and the expanded configuration.

As shown in FIGS. 34-36, the lockout mechanism 3824 can include a first window 3834 and a second window 3836. The first window 3834 is sized to receive the first arm 3830 of the leaf spring 3826 and the second window 3836 is sized to receive the second arm 3832 of the leaf spring 3826. In various embodiments, the leaf spring 3826 can be movable relative to the windows 3834, 3836 between an unlocked position (as shown in FIG. 35) and a lockout position (as shown in FIG. 36). In one aspect, as the leaf spring 3826 moves to the lockout position, the first arm 3830 can rotate into the first window 3834 and the second arm 3832 can rotate into the second window 3836. When the leaf spring 3826 is in the lockout position, the first arm 3830 can engage the first window 3834 and the second arm 3832 can engage the second window 3836, thereby preventing the leaf spring 3826 from moving back toward the unlocked position. In various embodiments, once the leaf spring 3826 is in the lockout position, the leaf spring 3826 is permanently locked in the lockout position. In various other embodiments, the leaf spring 3826 is capable of being reset to the unlocked position. In one example embodiment, a user can use their fingers to press the first arm 3830 and the second arm 3832 toward the central body 3828 through the first window 3834 and second window 3836, respectively, thereby allowing the leaf spring 3826 to move back to the unlocked position.

In various embodiments, the lockout mechanism 3824 can include a piston head 3838. The piston head 3838 can be movable to detect if a buttress layer 3840 is present within the end effector. In one example embodiment, as is shown in FIG. 35, in an instance where a buttress layer 3840 is present, the piston head 3838 can contact the buttress layer 3840. In another example embodiment, as is shown in FIG. 36, in an instance where a buttress layer in not present, the piston head 3838 can move beyond the position of where the buttress layer 3840 would be positioned. In such an instance, the piston head 3838 moving beyond the buttress layer 3840 location can cause the leaf spring 3826 to actuate and transition to the expanded configuration, thereby causing a lock-out situation, as will be described in more detail below. In various embodiments, when the lockout mechanism 3824 is in the lock-out situation, the firing member of the surgical instrument is prevented from performing a firing stroke.

Continuing to refer to FIGS. 35 and 36, the lockout mechanism 3824 can include a piston rod shaft 3842 and a piston rod cylinder 3844. In various embodiment, the piston rod shaft 3842 can be coupled to, or affixed, to the piston head 3838, with the distal end 3843 of the piston rod shaft 3842 terminating at the piston head 3838. In various embodiments, the piston rod cylinder 3844 can be coupled to, or affixed, to the leaf spring 3826, with the distal end 3845 of the piston rod cylinder 3844 terminating at the leaf spring 3826. In one aspect, the piston rod cylinder 3844 can be hollow and include an inside diameter that is greater than the outside diameter of the piston rod shaft 3842. In such an aspect, the piston rod shaft 3842 can be freely slidable within the piston cylinder 3844. In various embodiment, a spring 3846, such as a coil spring, can be coupled to the leaf spring 3826 and the piston head 3838, which can cause the piston rod shaft 3842 to be biased away from the piston rod cylinder 3844.

In one example operation when a buttress layer 3840 is present, as shown in FIG. 35, the piston head 3838 is pushed toward the buttress layer 3840 by way of the coil spring 3846. In one example embodiment, the piston head 3838 can be held in place prior to operation of the surgical instrument. Once the surgical instrument is actuated and a firing member is caused to begin moving within the end effector, the piston head 3838 can be released, initiating the lockout mechanism 3824. In one aspect, the coil spring 3846 is partially compressed such that, when the lockout mechanism 3838 is activated, the coil spring 3846 can force the piston head 3838 toward the position of the buttress layer 3840. In various embodiments, the spring leaf 3826 is held at least substantially in place, such as by friction fit or by the piston rod cylinder 3844, as examples, such that the leaf spring 3826 is prevented from moving upward and away from the position of the buttress layer 3840.

Continuing to refer to FIG. 35, as a buttress layer 3840 is present, the piston head 3838 abuts a surface of the buttress layer 3840 and halts further motion of the piston head 3838. Owing to the displacement of the piston head 3838 toward the buttress layer 3840, tension can be developed within the spring 3846, causing the coil spring 3846 to impart a force onto the leaf spring 3826, pulling the leaf spring 3826 toward the piston head 3838. As the leaf spring 3826 moves toward the buttress layer 3840, the coil spring 3846 begins to compress, owing to the piston head 3838 being held in place by the buttress layer 3840. As the coil spring 3846 compresses, the coil spring 3846 resistance force builds up and stops travel of the leaf spring 3826 before the first arm 3830 and the second arm 3832 are able to reach the first window 3834 and the second window 3836, respectively, preventing a lock-out situation. In one aspect, preventing the lock-out situation allows the firing member to freely to travel unobstructed through the end effector.

In another example operation when a buttress layer is absent, as shown in FIG. 36 the piston head 3838 is pushed toward the buttress layer 3840 by the coil spring 3846. In one aspect, the coil spring 3846 is partially compressed such that, when the lockout mechanism 3838 is activated, the coil spring 3846 can force the piston head 3838 toward the intended position of the buttress layer 3840, In various embodiments, the spring leaf 3826 is held at least substantially in place, such as by friction fit or by the piston rod cylinder 3844, as examples, such that the spring leaf 3826 is prevented from moving upward and away from the intended position of the buttress layer 3840.

Continuing to refer to FIG. 36, as a buttress layer is absent, the piston head 3838 moves beyond the intended position of where the buttress layer 3840 would be located. Owing to the displacement of the piston head 3838 toward the intended position of the buttress layer 3840, tension can be developed in the spring 3846, which causes the coil spring 3846 to impart a force onto the leaf spring 3826, pulling the leaf spring 3826 toward the piston head 3838. As the buttress layer 3840 is absent and the piston head 3838 is allowed to continue moving beyond the intended position of the buttress layer 3840, the coil spring 3846 does not compress at the same rate as if a buttress layer 3840 were present. As the coil spring 3846 is unable to generate enough coil resistance force to deter movement of the leaf spring 3826, the first arm 3830 and the second arm 3832 of the leaf spring 3826 are able to reach the first window 3834 and the second window 3836, respectively, and are therefore actuated to the expanded position, initiating a lock-out situation. In various embodiments, a lock-out situation can include preventing distal translation of a firing member through the end effector.

In various embodiments, the lockout mechanism 3824 can be made primarily out of plastic to enable elastic deformation to control the lockout. In various embodiments, the piston rod shaft 3842 and the piston rod cylinder 3844 can be comprised of metal to enhance rigidity, thereby allowing the lockout mechanism 3824 to resist side loading of the firing member and for connection to portions of the end effector, such as the anvil or channel body, as examples. The lockout mechanism 3824 can be placed in conjunction with other mechanisms to enable detection of proper buttress positioning throughout the entire area of the anvil or cartridge body. In one aspect, the lockout mechanism 3824 can be positioned to lockout motion of the firing that would lead to initial tissue clamping. In other aspects, the lockout mechanism 3824 can be positioned to lockout motion of the firing that would lead to firing of the staple cartridge within the end effector.

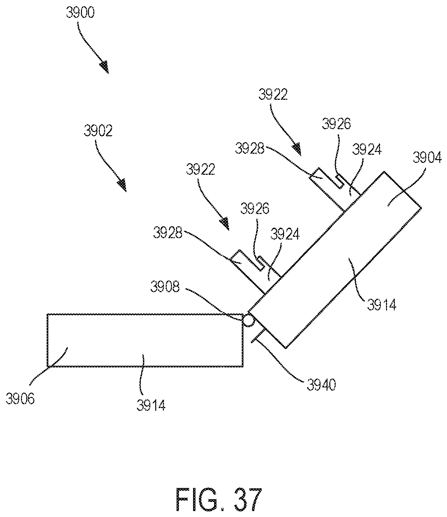

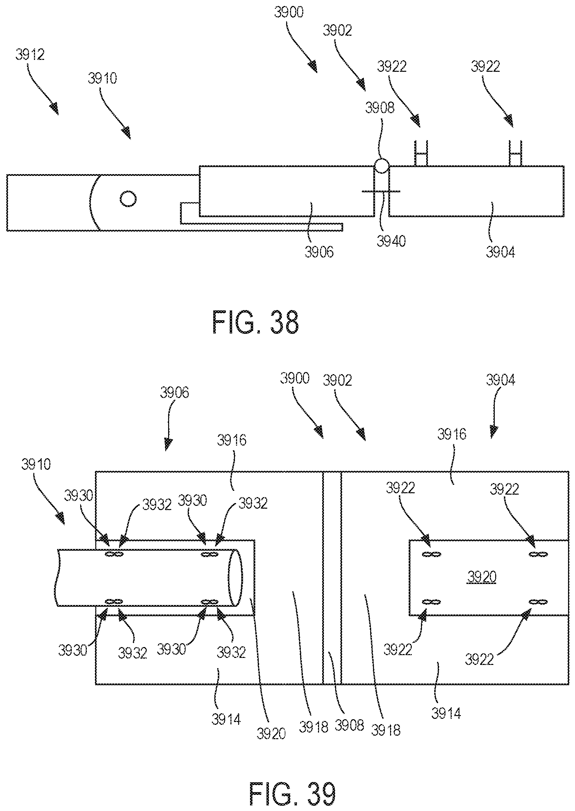

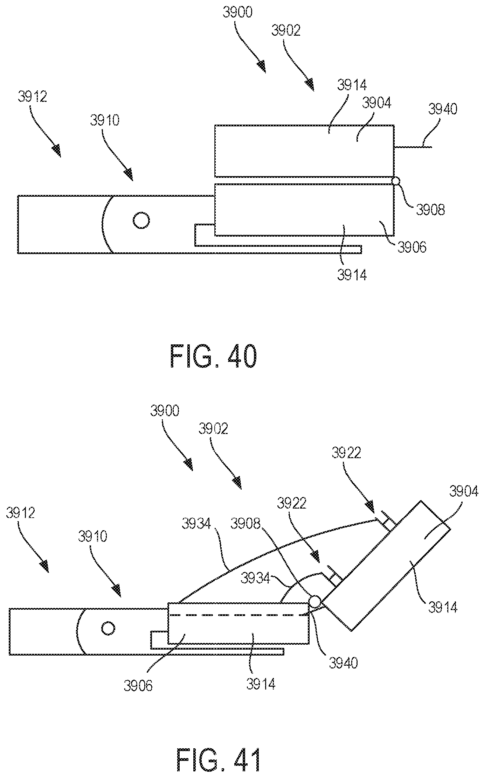

Referring now to FIG. 37, a suture applier 3900 is illustrated in accordance with at least one aspect of the present disclosure. The suture applier 3900 can include a housing assembly 3902 that includes a first housing half 3904 and a second housing half 3906 pivotably coupled to the first housing half 3904 about a pivot 3908. The first housing half 3904 can be rotatable relative to the second housing half 3906 between an open position (see FIG. 38) and a closed position (see FIG. 40) to capture an anvil 3910 of an end effector 3912 therebetween. Each of the first housing half 3904 and the second housing half 3906 can include a first leg 3914, a second leg 3916, a connector 3918 connecting the first leg 3914 to the second leg 3916, and surfaces 3920 (all shown most clearly in FIG. 39).

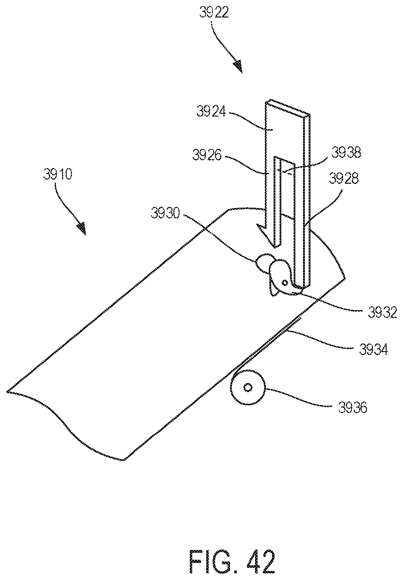

In various embodiments, the suture applier 3900 can include a plurality of plungers 3922 extending from the surface 3920 first housing half 3904. Referring to FIG. 42, each of the plungers 3922 can include a base 3924, a needle 3926, and a cam arm 3928 as will be described in more detail below.

In operation, the first housing half 3904 can be moved to the open position, as is shown in FIG. 38. The anvil 3910 can then be placed within the second housing half 3906 such that, when the first housing half 3904 is rotated to the closed positon, as is shown in FIG. 40, the anvil 3910 can be captured between the first housing half 3904 and the second housing half 3906. In various embodiments, the first leg 3914, the second leg 3916, and the connector 3918 of the second housing half 3906 are sized to guide the anvil 3910 such that, when a tissue contacting surface of the anvil 3910 abuts the surface 3920 of the second housing half 3906, apertures 3930 and cam members 3932 on the surface of the anvil 3910, which will be discussed in more detail below, are aligned with the needles 3926 and cam arms 3928 of the plungers 3922, respectively, when the first housing half 3904 is rotated to the closed position.

As shown in FIG. 39 and most clearly in FIG. 42, the anvil 3910 can include a plurality of apertures 3930 and cam members 3932 (FIG. 42 only illustrates one aperture 3930 and cam member 3932, but this is merely for illustrative purposes and it should be understood that the anvil 3910 can include a plurality of apertures 3930 and cam members 3932, each pair corresponding to a plunger 3922 in the first housing half 3904, as will be described in more detail below).

In various aspects, the apertures 3930 are sized to receive the needles 3926 of the plungers 3922 as the first housing half 3904 is rotated toward the closed position. The needles 3926 can travel through the apertures 3930 and extend into the second housing half 3906 as the first housing half 3904 is brought to the closed position. In the second housing half 3906, each of the needles 3926 can interface and capture a suture leg 3934. In one aspect, the second housing half 3906 can include a plurality of spools 3936 of suture material such that, when the needles 3926 extend into the second housing half 3906, the needles 3926 can interface and capture the free suture leg 3934 extending from spool 3936. Once the needle 3926 has coupled to and captured the suture leg 3934, the first housing half 3904 can be rotated toward the open position, causing the needles 3926 to pull the suture legs 3934 through the apertures 3930 of the anvil 3910 (shown in FIG. 41). In various embodiments, the suture legs 3634 can be coupled to a buttress layer such, as the suture legs 3934 are pulled through the apertures 3930, the buttress layer can be compressed against a tissue contacting surface of the anvil 3910.

As reference above, the anvil 3910 can include a plurality of cam members 3932. The cam members 3932 can be rotatably coupled to the anvil 3910 and can be rotatable between an engaged position and a disengaged position (disengaged position shown in FIG. 42). In various aspects, as the first housing half 3904 is rotated toward the closed position, the cam arms 3928 of the plungers 3922 can engage the cam member 3932 and rotate the cam member 3932 toward the disengaged position prior to the needle 3926 entering the aperture 3930. In one aspect, this can be accomplished by having the cam arm 3928 extend further from the base 3924 than the needle 3926, as is shown clearly in FIG. 42. As the needle 3926 traverses the aperture 3930 to interface with the suture legs 3934 in the second housing half 3906, as described above, the cam arm 3928 can ride along cam members 3932 to maintain the cam members 3932 in the disengaged position. In one example embodiment, the cam member 3932 can be sized such that the cam member 3932 can slide between the needle 3926 and the cam arm 3928 in the gap 3938 as the needle 3926 moves through the aperture 3930. As the cam member 3932 slides within the gap 3938, the cam member 3932 can abut the cam arm 3928, keeping the cam member 3932 in the disengaged position.

As the first housing half 3904 is rotated to the open position and the needles 3926 brings the suture legs 3934 through the apertures 3930, the cam arms 3928 can disengage the cam members 3932. In various embodiments, a biasing mechanism, such as a spring, can bias the cam member 3932 toward the engaged position such that, as the cam arms 3928 disengages the cam members 3932, the cam members 3932 can rotate towards the engaged position and engage the suture legs 3934 pulled through the apertures 3930 of the anvil 3910. In one aspect, the cam members 3932 can engage and hold the suture legs 3934, maintaining tension of the suture legs 3934 through the apertures 3930 of the anvil 3910.

Further to the above, as the first housing half 3904 rotates to the open position and the cam members 3932 engages the suture legs 3934, a knife member can sever the suture legs 3934 from the plungers 3922, leaving the suture legs 3934 engaged by the cam members 3932, and thus maintaining tension in the suture legs 3934 through the apertures 3930 of the anvil 3910. In one example embodiment, the knife can sever the suture legs 3934 as the first housing half 3904 approaches the open position. In one example embodiment, the first housing 3902 half can include an actuation feature 3940 extending from a pivot side thereof. As best shown in FIG. 41, as the first housing half 3904 moves to the open position, the actuation feature 3940 can rotate towards the second housing half 3906 and engage a knife positioned in the second housing half 3906. In one example embodiment, the second housing half 3906 can include an aperture defined in the connector 3918 of the second housing half 3906 such that the actuation feature 3940 can extend through the second housing half 3906 and engage the knife as the first housing half 3904 is rotated toward the open position. In one aspect, the actuation feature 3940 can engage and actuate the knife, causing the knife to sever each of the suture legs 3934 to release the suture legs 3914 from the plungers 3922, but still maintain the tension in the suture legs 3934 with the cam members 3932. In one example embodiment, the knife can progressively sever the suture legs 3934 are the first housing half 3904 is rotated toward the open position.

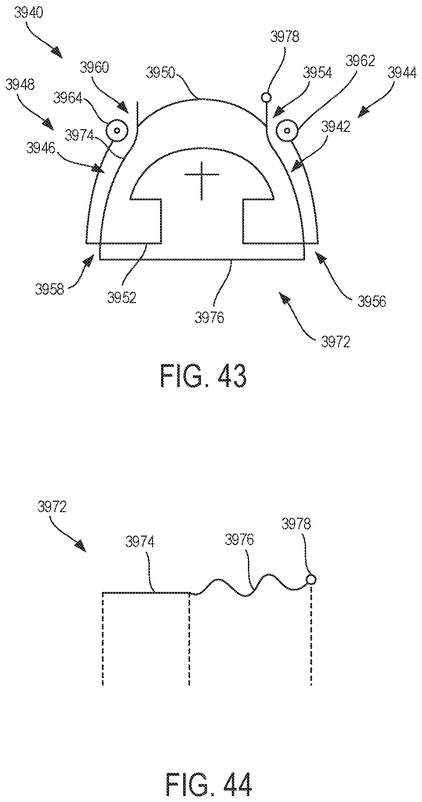

Referring now to FIG. 43, an anvil 3940 is provided in accordance with at least one aspect of the present disclosure. In various embodiments, the anvil 3940 can include a first track 3942 on a first lateral side 3944 of the anvil 3940 and a second track 3946 on a second lateral side 3948 of the anvil 3940. Each of the tracks 3942, 3944 are sized to allow for a suture assembly to pass from one side of the anvil, such as an outer, top surface 3950 of the anvil 3940, to another side of the anvil, such as the tissue contacting surface 3952 of the anvil 3940, as will be described in more detail below. In one aspect, the first track 3942 can include an entrance aperture 3954 defined in the outer surface 3950 of the anvil 3940 and an exit aperture 3956 defined in the tissue contacting surface 3952 of the anvil 3940. Similarly, the second track 3946 can include an entrance aperture 3958 defined in the tissue contacting surface 3952 of the anvil 3940 and an exit aperture 3960 defined in the outer surface 3950 of the anvil 3940.

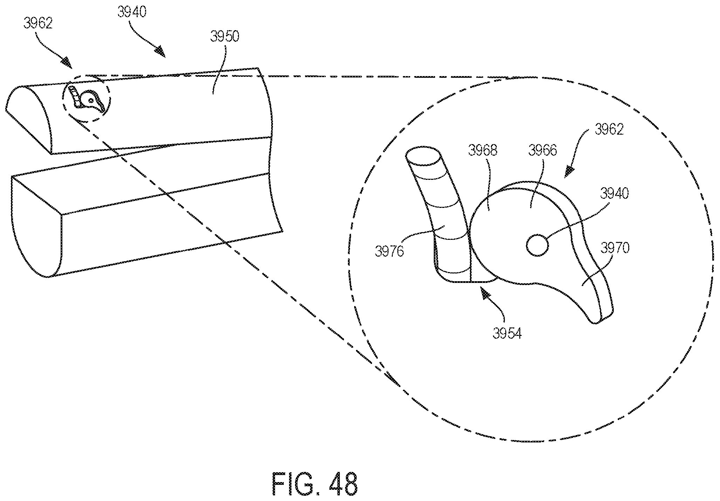

In various embodiments, the anvil 3940 can further include a first cam lock 3962 and a second cam lock 3964. Referring to FIG. 48, a detailed view of cam lock 3962 is provided, however, it should be understood that the second cam lock 3964 is of similar construction. As shown in FIG. 48, each of the cam locks 3962, 3964 can include a body portion 3966, an engagement surface 3968 extending from the body portion 3966, and a cam arm 3970 extending from the body portion 3966. In one aspect, the body portions 3966 of the cam locks 3962, 3964 can be rotatable coupled to the outer surface 3950 of the anvil 3940 by pins. In one aspect, the first cam lock 3962 can be rotatably coupled to the anvil 3940 near the entrance aperture 3954 of the first track 3942 and the second cam lock 3964 can be rotatable coupled to the anvil 3940 near the exit aperture 3960 of the second track 3946.

In one aspect, the cam locks 3962, 3964 can be rotatable relative to the anvil 3940 between a locked position and an unlocked position. In various embodiments, when the first and second cam locks 3962, 3964 are in the unlocked positions, the engagement surfaces 3968 of the cam locks 3962, 3964 are rotated away from their respective apertures 3954, 3960 defined in the outer surface 3950 of the anvil 3940, therefore allowing a suture assembly to pass through the respective first track 3942 and the second track 3946 uninterrupted. In addition, when the first and second cam locks 3962, 3964 are in the locked positions, the engagement surfaces 3968 of the cam locks 3962, 3964 can at least partially extend over their respective apertures 3954, 3960 defined in the outer surface 3950 of the anvil 3940 such that a suture extending through the respective aperture can be held in place by the engagement surfaces 3968 of the cam locks 3962, 3964. In various embodiments, the cam locks 3962, 3964 can be coupled to a biasing member, such as a torsional spring, such that the cam locks 3962, 3964 can be biased to the locked position. In one example embodiment, in order to rotate the cam locks 3962, 3964 to the unlocked position, a force can be applied to the cam arms 3970, causing the cam locks 3962, 3964 to rotate about pins to the unlocked positon.

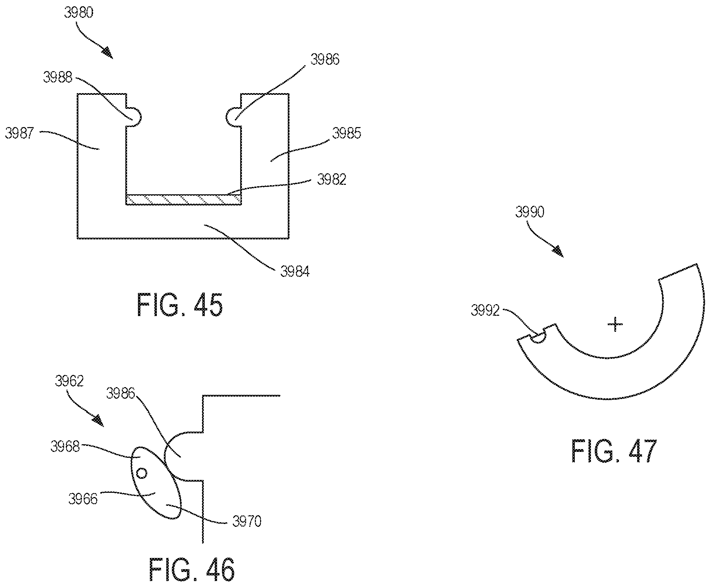

In various embodiments, referring to FIGS. 43 and 44, a suture assembly 3972 can be usable with the anvil 3940 to attach a buttress layer to the tissue contacting surface 3952 of the anvil 3940. In various embodiments, the suture assembly 3972 can include a semi-rigid, flexible needle 3974, a suture 3976 removably coupled to and extending from the needle 3974, and a hard stop ball 3978 coupled to and extending from the suture 3976. The needle 3974 can be made of any suitable material, such as plastic, such that the needle 3974 is rigid enough to be threaded through the first and second tracks 3942, 3946 of the anvil 3940, while also being flexible enough to navigate any twists or turns in the tracks. In one example embodiment, the needle 3974 can include a sharp tip such that the needle 3974 can be threaded through a buttress layer, therefore coupling the suture assembly 3972 to the buttress layer. In another example embodiments, the needle 3974 can include a blunt tip in instances where the needle 3974 is intended to be wrapped around and support a bottom surface of the buttress layer, as opposed to piercing the buttress layer itself. In various embodiments, referring to FIG. 47, the needle can comprise a hooked shaped needle 3990 that can be utilized to facilitate passage of the needle 3990 through the first track 3942 and the second track 3946. In various embodiments, the hooked shaped needle can include a coupling portion 3992 that can couple to the suture 3976 of the suture assembly 3972.

In one example operation, the anvil 3940 can be placed in a buttress cartridge 3980, illustrated in FIG. 45, to apply a buttress layer 3982 to the anvil 3940. The buttress cartridge 3980 can include a base 3984 including a buttress layer 3982 positioned thereon, a first sidewall 3985 extending from the base 3984 and a second sidewall 3987 extending from the base 3984. The sidewalls 3985, 3987 can be sized such that, when the tissue contacting surface 3952 is brought towards the base 3984 of the buttress cartridge 3980, the sidewalls 3985, 3987 can force the anvil 3940 into proper lateral alignment with the base 3984 to avoid the buttress layer 3982 being mispositioned on the tissue contacting surface 3952.

In various embodiments, the buttress cartridge 3980 can further include a first arm 3986 extending from the first sidewall 3985 and a second arm 3988 extending from the second sidewall 3987. The first and second arms 3986, 3988 can be sized that, as the tissue contacting surface 3952 is moved towards the buttress layer 3982 in the buttress cartridge 3980, the first and second arms 3986, 3988 can contact the first and second cam arms 3970 of the first and second cam locks 3962, 3964, respectively, causing the first and second cam locks 3962, 3964 to rotate to the unlocked positions. An example of this procedure is illustrated in FIG. 46. In one aspect, the first and second arms 3986, 3988 can hold the cam locks 3962, 3964 in the unlocked positions until the anvil 3940 is moved out of the buttress cartridge 3980, at which point the biasing members can rotate the cam members 3962, 3964 back to their locked positions.

In one example embodiment, the tissue contacting surface 3952 can be moved into the buttress cartridge 3980 and into contact the buttress layer 3982 on the base 3984. In various embodiments, buttress layer 3982 can include an adhesive that can at least partially adhere the buttress layer 3982 to the tissue contacting surface 3982. As the tissue contacting surface 3952 of the anvil 3940 is brought into contact with the buttress layer 3982, the first and second arms 3986, 3988 can move and hold the cam locks 3962, 3964 in the unlocked position. In various embodiments, the suture assembly 3972 can then be utilized to further couple the buttress layer 3982 to the anvil 3970 in a manner as was described above. In one example embodiment, while the cam locks 3962, 3964 are held in the unlocked position, the needle 3974 can be threaded from the entrance aperture 3954 to the exit aperture 3964 of the first track 3942, coupled to the buttress layer 3982 in any suitable manner (such as the manners described above), and then threaded from the entrance aperture 3958 to the exit aperture 3960 of the second track 3946. In one example embodiment, the base 3984 can include a track defined therein that includes entrance and exit apertures that correspond to the exit aperture 3956 and the entrance aperture 3958, respectively, such that the needle 3974 can travel through the first track 3942, through (or around) the buttress layer 3982, through the track in the base 3984, back through (or around) the buttress layer 3982 and through the second track 3946.

In various embodiments, as the needle 3970 is pulled through the exit aperture 3956 of the second track 3946, the hard stop ball 3978 can abut the outer surface 3950 of the anvil 3940. In one aspect, the hard stop ball 3978 can be sized such that the hard stop ball 3978 is prevented from entering the entrance aperture 3954 of the first track 3942, therefore preventing the suture assembly 3972 from being pulled completely through the first track 3942. In various embodiments, the suture 3976 can have a sufficient length so as to allow the needle 3974 to be pulled through the exit aperture 3960 prior to the hard stop ball 3978 contacting the entrance aperture 3954, therefore allowing a user to pull the needle 3974 and tension the suture 3976, causing the buttress layer 3982 to be securely pulled against the tissue contacting surface 3952 of the anvil 3940. In various embodiments, the above-described threading procedure can clear old suture material that is still held in the tracks 3942, 3946 of the anvil 3940 from previous uses of the anvil 3940.

In one aspect, once the buttress layer 3982 is coupled to the anvil 3940 by way of the suture assembly 3972 and the suture 3976 has been sufficiently tensioned by way of the needle 3974 and the hard stop ball 3978, the anvil 3940 can be moved out of the buttress cartridge 3980. Movement of the anvil 3940 away from the buttress cartridge 3940 can cause the cam locks 3962, 3964 to rotate towards their locked positions, therefore causing the engagement surfaces 3968 of the cam locks 3962, 3964 to engage portions of the suture 3976 extending from the outer surface 3950 of the anvil 3940 (at the entrance aperture 3954 of the first track 3942 and the exit aperture 3960 of the second track 3946), holding the suture assembly 3972 in place and maintaining tension in the suture 3976. In various embodiments, once the cam locks 3962, 3964 have been rotated to the locked positions, the needle 3974 of the suture assembly 3972 can be decoupled from the suture 3976, allowing the needle to be used with a different suture assembly 3972.

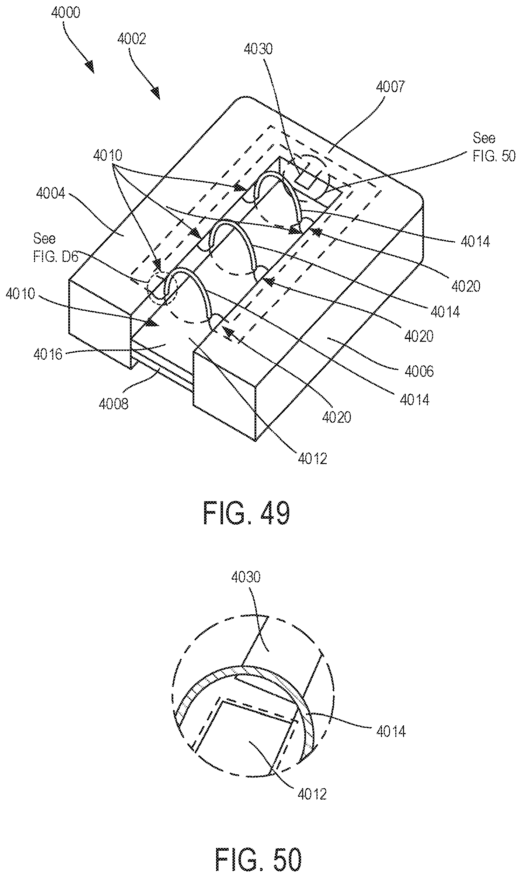

Referring now to FIG. 49, a buttress applier cartridge 4000 is provided in accordance with at least one aspect of the present disclosure. The buttress applier cartridge 4000 can include a generally U-shaped housing assembly 4002 that includes a first leg 4004, a second leg 4006, and a connector 4007 connecting the first leg 4004 and the second leg 4006. The housing assembly 4002 can further include a support platform 4008 that can support a buttress assembly 4010 thereon. In various embodiments, the housing assembly 4002 can be of similar constriction to other buttress applier cartridges described herein, such as buttress applier cartridges 3200, 3300, as examples.

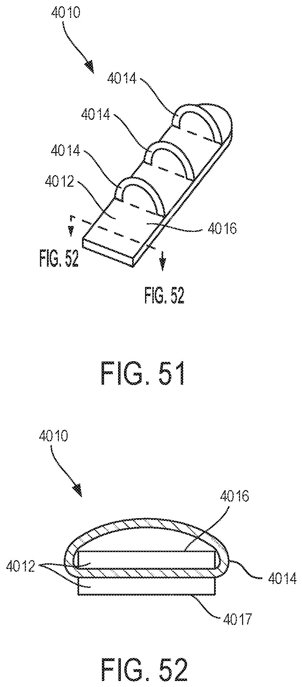

In one aspect, the buttress applier cartridge 4000 can be utilized to apply the buttress assembly 4010 to an anvil of an end effector. In various embodiments, referring to FIG. 51, the buttress assembly 4010 can include a buttress layer 4012 and a plurality of suture loops 4014 extending from the buttress layer 4012. In various embodiments, as shown in FIG. 52, the suture loops 4014 can be embedded in the buttress layer 4012 between a top surface 4016 of the buttress layer 4012 and a bottom surface 4017 of the buttress layer 4012. In other embodiments, the suture loops 4014 can be coupled to the buttress layer 4012 in any suitable manner such that the suture loops 4014 can support the buttress layer 4012 as the buttress layer 4012 is applied to an anvil. In one such embodiment, the suture loops 4014 are not continuous loops as is shown in FIG. 52, rather, the legs of the suture loops 4014 are attached the buttress layer 4012 at discrete locations, as described elsewhere herein. In various other embodiments, the suture loops 4014 are not embedded between a single buttress layer 4012, rather, a portion of the suture loop 4014 is capture between two pieces of buttress layer 4012 adhered together.

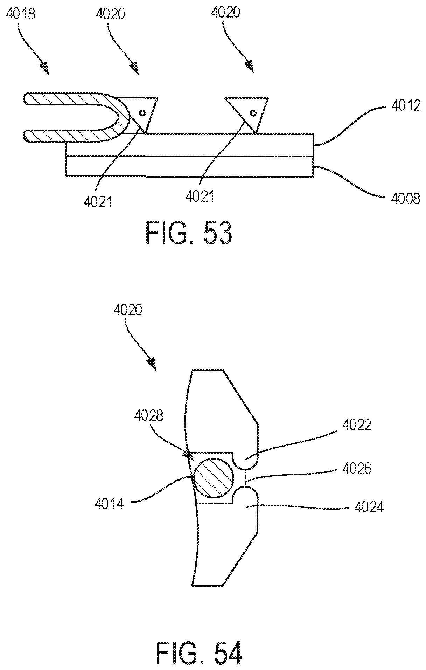

As shown in FIG. 49, the buttress assembly 4010 can be positioned within the buttress applier cartridge 4000 and can be supported by the support platform 4008. In one aspect, the buttress applier cartridge 4000 can include a plurality of wedge shaped suture clamps 4020 extending from the first leg 4004 and the second leg 4006 of the buttress applier cartridge 4000. In various embodiments, referring to FIG. 54, the suture clamps 4020 can include a first arm 4022 and a second arm 4024 spaced from the first arm 4022 so as to define a gap 4026 therebetween such that, when the buttress assembly 4010 is properly seated on the support platform 4008, the legs of the suture loops 4014 can be held in a recess 4028 by the first arm 4022 and the second arm 4024. In other embodiments, the legs of the suture loops 4014 can be positioned in the recesses 4028 of the suture clamps when the suture clamps 4020 are in a resting state, as will be described in more detail below.

In various embodiments, the suture clamps 4020 can be transitionable between a resting state, where the suture clamps 4020 can hold the legs of the suture loops 4014 within the recesses 4028, and an actuated state, where the suture clamps 4020 can allow the legs of the suture loops 4014 to escape the suture clamps 4020. In one embodiment, a portion of the suture clamps 4020 can move toward the connector 4007 while another portion of the suture clamps 4020 can remain stationary. In such an embodiment, the relative movement between the portions of the suture clamps 4020 can transition the suture clamps 4020 between the resting state and the actuated state. As the suture clamps 4020 move to the actuated state, as described above, the legs of the suture loops 4014 can be released and allowed to move out of the suture clamps 4020. In various other embodiments, one or both of the first arm 4022 and the second arm 4024 of the suture clamps 4020 can be moveable relative to the other to increase the gap size 4026 therebetween. In various embodiments, the relative movement of the first arm 4022 and the second arm 4024 can transition the suture clamps 4020 between the resting state and the actuated state.

In operation, a user can slide an anvil 4018 from the open end of the buttress applier cartridge 4000 (the end opposite of the connector 4007) along the buttress assembly 4010 toward the connector 4007. In one aspect, as shown in FIG. 53, as the anvil 4018 moves under the proximal-most suture loop 4014, the anvil 4018 can abut a camming surface 4021 of the proximal-most suture clamps 4020, causing the suture clamps 4020 to slightly elevate, causing the suture loop 4014 held by the suture clamp 4020 to expand in tension. The anvil 4018 can continue to progress along the buttress applier cartridge 4000 and through the suture loops 4014 and contact the camming surfaces 4021, causing the suture clamps 4020 to slightly elevate and develop tension in all of the suture loops 4014. In various embodiments, the connector 4007 of the housing assembly 4002 can include a release button 4030 operably coupled to the suture clamps 4020. In one aspect, after the anvil 4018 has progressed through all of the suture loops 4014 and reached the connector 4007, the anvil 4018 can engage the release button 4030, causing the suture clamps 4020 to transition to the actuated state, releasing the suture loops 4014 from the suture clamps 4020, as described above. As the suture loops 4014 were tensioned as the anvil 4018 moved along the buttress assembly 4010 towards the release button 4030 (owing to the elevation of the suture clamps 4020 by way of camming surfaces 4021), the suture loops 4014 can be released from the suture clamps 4020 and tighten around the anvil 4018, coupling the buttress assembly 4010 to the anvil 4018, and more specifically, coupling the buttress layer 4012 to a tissue contacting surface of the anvil 4018.

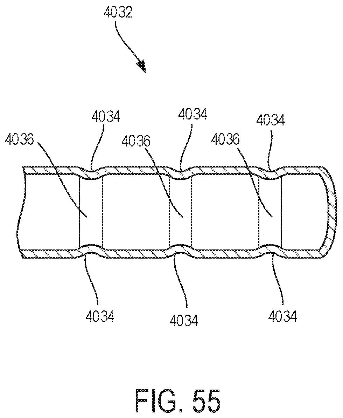

In various embodiments, as shown in FIG. 55, an anvil 4032 is provided that can be utilized with the buttress applier cartridge 4000. The anvil 4032 includes a plurality of detents 4034 along the length thereof that can receive the suture loops 4014 when the buttress assembly 4010 is coupled to the anvil 4032. The detents 4034 can be sized to receive and hold the suture loops 4014 such that the chance of the suture loops 4014 sliding along the anvil 4032 is minimized. In various other embodiments, the anvil 4032 can include a plurality of grooves 4036 extending between laterally offset detents 4034 that can further be utilized to maintain the suture loops 4014 on the anvil 4032.

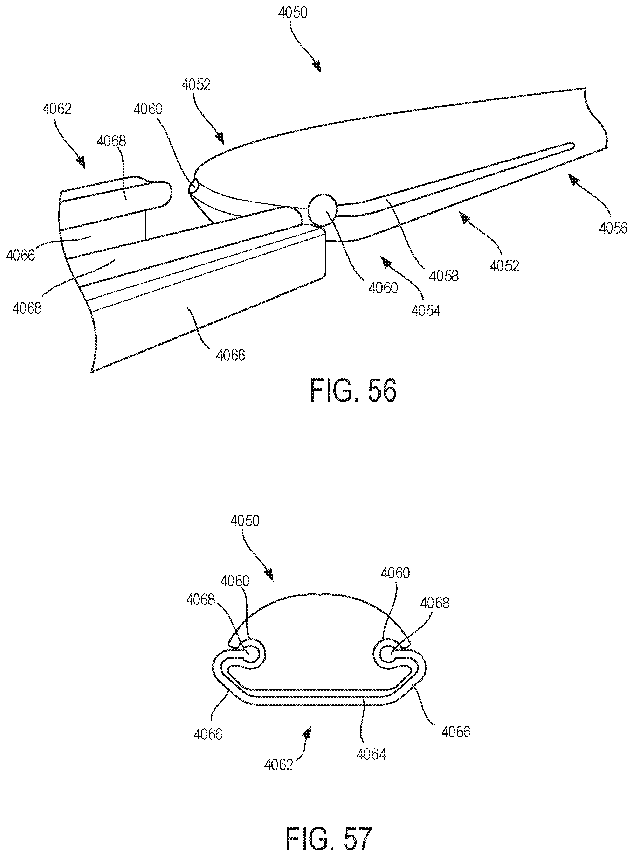

Referring now to FIG. 56, an anvil 4050 is provided in accordance with at least one aspect of the present disclosure. The anvil 4050 can include a set of tracks 4052 defined in both lateral sides of the anvil 4050 that extend along the length thereof from a receiving location 4054 to an ending location 4056. The tracks 4052 can include a narrow track 4058 and an expanded receiving aperture 4060 that can be larger in size than the narrow track 4058. In one aspect, the aperture 4060 can correspond to the receiving location 4054 and an end of the narrow track 4058 can correspond to the ending location 4056.

In various embodiments, the anvil 4050 can interface with a buttress assembly 4062. Referring to FIGS. 56 and 57, the buttress assembly 4062 can include a buttress layer 4064, arms 4066 extending from the buttress layer 4064, and a stop 4068 extending from each arm 4066. The stops 4068 are sized to be received within the receiving apertures 4060, while the arms 4066 are sized such that a portion thereof can be slidably received within the narrow tracks 4058. In one aspect, the buttress assembly 4062 can be coupled to the anvil 4050 by sliding the stops 4068 and the arms 4066 within the receiving aperture 4060 and the narrow tracks 4058, respectively. Once coupled, the size of the stops 4068 can prevent the stops 4068 from escaping laterally through the narrow tracks 4058, maintaining the buttress assembly 4062 coupled to the anvil 4050. In various embodiments, the arms 4066 and the stops 4068 are manufactured of a semi-rigid material to prevent the arms 4066 and stops 4068 from releasing from the anvil 4050 through the narrow tracks 4058. In various embodiments, only the stops 4068 are manufactured of a semi-rigid material so as to prevent the stops 4068 from escaping through the narrow tracks 4058.

In operation, the anvil 4050 can be coupled to the buttress assembly 4062 in the manner described above. Once coupled, the anvil 4050 has been utilized in a stapling procedure as described elsewhere here, resulting in the buttress layer 4064 being stapled to tissue. To remove the stapled buttress assembly 4062 from the anvil 4050, the anvil 4050 can be pulled proximally so that the stops 4068 and the arms 4066 of the buttress assembly 4062 can slide through the tracks 4052 and be released from the anvil 4050 through the receiving apertures 4060 and the narrow tracks 4058, respectively. As substantially all of the buttress assembly 4062 is left at the stapling site, there are no post-firing steps regarding the buttress assembly 4062, and therefore, a new buttress assembly can be coupled to the anvil 4050 in the same manner as described above. The above-provided design eliminates the need for another other type of buttress applicator/system and eliminates the need for sutures.

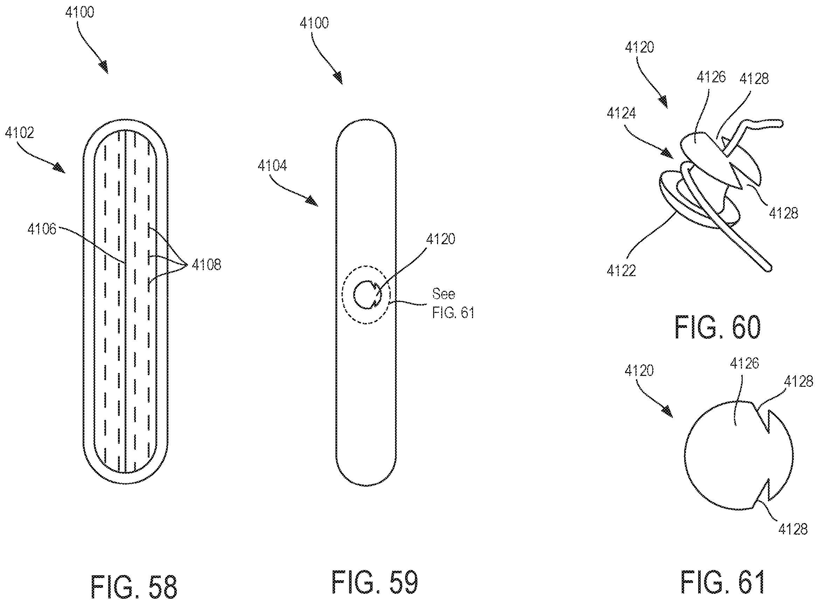

Referring now to FIGS. 58 and 59, an anvil 4100 is provided in accordance with at least aspect of the present disclosure. The anvil 4100 can include a tissue contacting surface 4102 and an outer surface 4104 on an opposite to the tissue contacting surface 4102. The tissue contacting surface 4102 can include an elongate slot 4106 and a plurality of staple pockets 4108 (only three are pointed to). The outer surface 4104 can include a suture lock 4120 extending therefrom. In various embodiments, as shown best in FIG. 60, the suture lock 4120 can include a base 4122 coupled to the outer surface 4104, a receiving area 4124 extending from the base 4122, and a cap 4126 extending from the receiving area 4124. In various embodiments, the cap 4126 can have a larger diameter than the receiving area 4124 such that the cap 4126 and the receiving area 4124 define a 'mushroom-like' shape, as shown in FIG. 60. In various embodiments, as shown in FIGS. 60 and 61, the cap 4126 can include a plurality of detents 4128 defined therein that can receive and hold suture legs (one such example shown in FIG. 60), as will be described in more detail below. In one embodiment, as is shown in FIGS. 60 and 61, the cap 4126 can include a pair of opposing detents 4128.

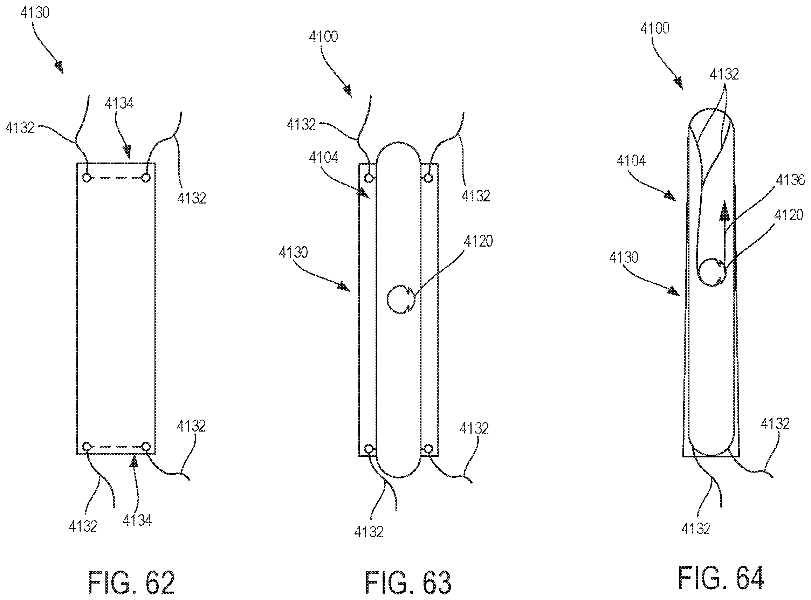

In various embodiments, the anvil 4100 can interface with a buttress later 4130 including a plurality of suture legs 4132. In one embodiment, as is shown in FIG. 62, the buttress layer 4130 can include four suture legs 4132 extending from the four corners of the buttress layer 4130. In one aspect, a pair of suture legs 4132 can be part of one continuous suture 4134 that threads through one corner of the buttress layer 4130 and out of another corner of the buttress layer 4130. In various other embodiments, the suture legs 4132 can be discretely coupled to corners of the buttress layer 4130. Other embodiments are envisioned where the suture legs 4132 extend from the buttress layer at other locations other than the corners of the buttress layer 4130, such as the sides of the buttress layer 4130.

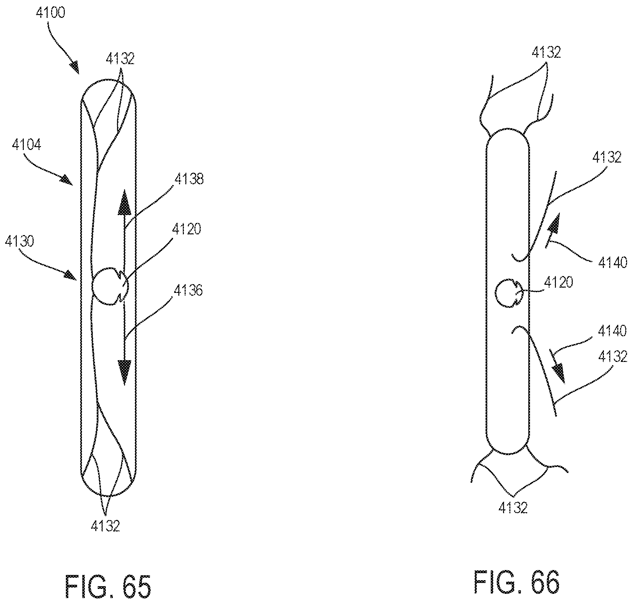

As shown in FIG. 63, the buttress layer 4130 can interface with the tissue contacting surface 4102 of the anvil 4100 such that the suture legs 4132 are laterally positioned away from the anvil 4100. In one aspect, as shown in FIG. 64, a first pair of suture legs can be pulled 4136 around the receiving area 4124 of the suture lock 4120 and held by a first detent 4128 defined in the cap 4126 of the suture lock 4120. In addition, as shown in FIG. 65, a second pair of suture legs 4132 can be pulled 4138 around the receiving area 4124 of the suture lock 4120 and held by a second detent 4128 defined in the cap 4126 of the suture lock 4120. The pairs of suture legs 4132 can be held by the suture lock 4120, thus coupling the buttress layer 4130 to the anvil 4100, allowing the anvil 4100 to be used in a stapling procedure as described elsewhere herein.

After the completion of the stapling procedure, the free ends of the suture legs 4132 extending from the cap 4126 of the suture lock 4120 can be pulled 4140, as shown by arrows in FIG. 66. The detents 4128 of the cap 4126 can include a sharp edge such that, as the free ends of the suture legs 4132 are pulled 4140, the detents 4128 can sever the suture legs 4132, releasing the buttress layer 4130 from the anvil 4100.

While various of the above-provided embodiments are discussed in regards to performing action on an anvil, such as the foregoing description regarding FIGS. 58-66 which generally discusses coupling a buttress layer to an anvil, it should be understood that the embodiments can be modified or utilized with an elongate channel and/or a staple cartridge in the alternative, or in combination therewith.

Numerous specific details are set forth to provide a thorough understanding of the overall structure, function, manufacture, and use of the embodiments as described in the specification and illustrated in the accompanying drawings. Well-known operations, components, and elements have not been described in detail so as not to obscure the embodiments described in the specification. The reader will understand that the embodiments described and illustrated herein are non-limiting examples, and thus it can be appreciated that the specific structural and functional details disclosed herein may be representative and illustrative. Variations and changes thereto may be made without departing from the scope of the claims.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a surgical system, device, or apparatus that "comprises," "has," "includes" or "contains" one or more elements possesses those one or more elements, but is not limited to possessing only those one or more elements. Likewise, an element of a system, device, or apparatus that "comprises," "has," "includes" or "contains" one or more features possesses those one or more features, but is not limited to possessing only those one or more features.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, the reader will readily appreciate that the various methods and devices disclosed herein can be used in numerous surgical procedures and applications including, for example, in connection with open surgical procedures. As the present Detailed Description proceeds, the reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongate shaft of a surgical instrument can be advanced.

A surgical stapling system can comprise a shaft and an end effector extending from the shaft. The end effector comprises a first jaw and a second jaw. The first jaw comprises a staple cartridge. The staple cartridge is insertable into and removable from the first jaw; however, other embodiments are envisioned in which a staple cartridge is not removable from, or at least readily replaceable from, the first jaw. The second jaw comprises an anvil configured to deform staples ejected from the staple cartridge. The second jaw is pivotable relative to the first jaw about a closure axis; however, other embodiments are envisioned in which the first jaw is pivotable relative to the second jaw. The surgical stapling system further comprises an articulation joint configured to permit the end effector to be rotated, or articulated, relative to the shaft. The end effector is rotatable about an articulation axis extending through the articulation joint. Other embodiments are envisioned which do not include an articulation joint.

The staple cartridge comprises a cartridge body. The cartridge body includes a proximal end, a distal end, and a deck extending between the proximal end and the distal end. In use, the staple cartridge is positioned on a first side of the tissue to be stapled and the anvil is positioned on a second side of the tissue. The anvil is moved toward the staple cartridge to compress and clamp the tissue against the deck. Thereafter, staples removably stored in the cartridge body can be deployed into the tissue. The cartridge body includes staple cavities defined therein wherein staples are removably stored in the staple cavities. The staple cavities are arranged in six longitudinal rows. Three rows of staple cavities are positioned on a first side of a longitudinal slot and three rows of staple cavities are positioned on a second side of the longitudinal slot. Other arrangements of staple cavities and staples may be possible.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions by a sled. The sled is movable between a proximal position adjacent the proximal end and a distal position adjacent the distal end. The sled comprises a plurality of ramped surfaces configured to slide under the drivers and lift the drivers, and the staples supported thereon, toward the anvil.

Further to the above, the sled is moved distally by a firing member. The firing member is configured to contact the sled and push the sled toward the distal end. The longitudinal slot defined in the cartridge body is configured to receive the firing member. The anvil also includes a slot configured to receive the firing member. The firing member further comprises a first cam which engages the first jaw and a second cam which engages the second jaw. As the firing member is advanced distally, the first cam and the second cam can control the distance, or tissue gap, between the deck of the staple cartridge and the anvil. The firing member also comprises a knife configured to incise the tissue captured intermediate the staple cartridge and the anvil. It is desirable for the knife to be positioned at least partially proximal to the ramped surfaces such that the staples are ejected ahead of the knife.

Although various devices have been described herein in connection with certain embodiments, modifications and variations to those embodiments may be implemented. Particular features, structures, or characteristics may be combined in any suitable manner in one or more embodiments. Thus, the particular features, structures, or characteristics illustrated or described in connection with one embodiment may be combined in whole or in part, with the features, structures or characteristics of one or more other embodiments without limitation. Also, where materials are disclosed for certain components, other materials may be used. Furthermore, according to various embodiments, a single component may be replaced by multiple components, and multiple components may be replaced by a single component, to perform a given function or functions. The foregoing description and following claims are intended to cover all such modification and variations.

The devices disclosed herein can be designed to be disposed of after a single use, or they can be designed to be used multiple times. In either case, however, a device can be reconditioned for reuse after at least one use. Reconditioning can include any combination of the steps including, but not limited to, the disassembly of the device, followed by cleaning or replacement of particular pieces of the device, and subsequent reassembly of the device. In particular, a reconditioning facility and/or surgical team can disassemble a device and, after cleaning and/or replacing particular parts of the device, the device can be reassembled for subsequent use. Those skilled in the art will appreciate that reconditioning of a device can utilize a variety of techniques for disassembly, cleaning/replacement, and reassembly. Use of such techniques, and the resulting reconditioned device, are all within the scope of the present application.

The devices disclosed herein may be processed before surgery. First, a new or used instrument may be obtained and, when necessary, cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, and/or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta radiation, gamma radiation, ethylene oxide, plasma peroxide, and/or steam.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, and/or examples can be implemented, individually and/or collectively, by a wide range of hardware, software, firmware, or virtually any combination thereof. Those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as one or more program products in a variety of forms, and that an illustrative form of the subject matter described herein applies regardless of the particular type of signal bearing medium used to actually carry out the distribution.

Instructions used to program logic to perform various disclosed aspects can be stored within a memory in the system, such as dynamic random access memory (DRAM), cache, flash memory, or other storage. Furthermore, the instructions can be distributed via a network or by way of other computer readable media. Thus a machine-readable medium may include any mechanism for storing or transmitting information in a form readable by a machine (e.g., a computer), but is not limited to, floppy diskettes, optical disks, compact disc, read-only memory (CD-ROMs), and magneto-optical disks, read-only memory (ROMs), random access memory (RAM), erasable programmable read-only memory (EPROM), electrically erasable programmable read-only memory (EEPROM), magnetic or optical cards, flash memory, or a tangible, machine-readable storage used in the transmission of information over the Internet via electrical, optical, acoustical or other forms of propagated signals (e.g., carrier waves, infrared signals, digital signals, etc.). Accordingly, the non-transitory computer-readable medium includes any type of tangible machine-readable medium suitable for storing or transmitting electronic instructions or information in a form readable by a machine (e.g., a computer).

As used in any aspect herein, the term "control circuit" may refer to, for example, hardwired circuitry, programmable circuitry (e.g., a computer processor including one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, smart phones, etc. Accordingly, as used herein "control circuit" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

As used in any aspect herein, the term "logic" may refer to an app, software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices.

As used in any aspect herein, the terms "component," "system," "module" and the like can refer to a computer-related entity, either hardware, a combination of hardware and software, software, or software in execution.

As used in any aspect herein, an "algorithm" refers to a self-consistent sequence of steps leading to a desired result, where a "step" refers to a manipulation of physical quantities and/or logic states which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities and/or states.

A network may include a packet switched network. The communication devices may be capable of communicating with each other using a selected packet switched network communications protocol. One example communications protocol may include an Ethernet communications protocol which may be capable permitting communication using a Transmission Control Protocol/Internet Protocol (TCP/IP). The Ethernet protocol may comply or be compatible with the Ethernet standard published by the Institute of Electrical and Electronics Engineers (IEEE) titled "IEEE 802.3 Standard", published in December, 2008 and/or later versions of this standard. Alternatively or additionally, the communication devices may be capable of communicating with each other using an X.25 communications protocol. The X.25 communications protocol may comply or be compatible with a standard promulgated by the International Telecommunication Union-Telecommunication Standardization Sector (ITU-T). Alternatively or additionally, the communication devices may be capable of communicating with each other using a frame relay communications protocol. The frame relay communications protocol may comply or be compatible with a standard promulgated by Consultative Committee for International Telegraph and Telephone (CCITT) and/or the American National Standards Institute (ANSI). Alternatively or additionally, the transceivers may be capable of communicating with each other using an Asynchronous Transfer Mode (ATM) communications protocol. The ATM communications protocol may comply or be compatible with an ATM standard published by the ATM Forum titled "ATM-MPLS Network Interworking 2.0" published August 2001, and/or later versions of this standard. Of course, different and/or after-developed connection-oriented network communication protocols are equally contemplated herein.

Unless specifically stated otherwise as apparent from the foregoing disclosure, it is appreciated that, throughout the foregoing disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order. Also, although various operational flow diagrams are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the particular features, structures or characteristics may be combined in any suitable manner in one or more aspects.

As discussed above, the surgical instruments disclosed herein may comprise control systems. Each of the control systems can comprise a circuit board having one or more processors and/or memory devices. Among other things, the control systems are configured to store sensor data, for example. They are also configured to store data which identifies the type of staple cartridge attached to a stapling instrument, for example. More specifically, the type of staple cartridge can be identified when attached to the stapling instrument by the sensors and the sensor data can be stored in the control system. This information can be obtained by the control system to assess whether or not the staple cartridge is suitable for use.

The surgical instrument systems described herein are motivated by an electric motor; however, the surgical instrument systems described herein can be motivated in any suitable manner. In certain instances, the motors disclosed herein may comprise a portion or portions of a robotically controlled system. U.S. Patent Application Serial No. 13/118,241, entitled SURGICAL STAPLING INSTRUMENTS WITH ROTATABLE STAPLE DEPLOYMENT ARRANGEMENTS, now U.S. Patent No. 9,072,535, for example, discloses several examples of a robotic surgical instrument system in greater detail. The disclosures of International Patent Publication No. WO 2017/083125, entitled STAPLER WITH COMPOSITE CARDAN AND SCREW DRIVE, published May 18, 2017, International Patent Publication No. WO 2017/083126, entitled STAPLE PUSHER WITH LOST MOTION BETWEEN RAMPS, published May 18, 2017, International Patent Publication No. WO 2015/153642, entitled SURGICAL INSTRUMENT WITH SHIFTABLE TRANSMISSION, published October 8, 2015, U.S. Patent Application Publication No. 2017/0265954, filed March 17, 2017, entitled STAPLER WITH CABLE-DRIVEN ADVANCEABLE CLAMPING ELEMENT AND DUAL DISTAL PULLEYS, U.S. Patent Application Publication No. 2017/0265865, filed February 15, 2017, entitled STAPLER WITH CABLE-DRIVEN ADVANCEABLE CLAMPING ELEMENT AND DISTAL PULLEY, and U.S. Patent Publication No. 2017/0290586, entitled STAPLING CARTRIDGE, filed on March 29, 2017, are referenced in their entireties.

The term "substantially", "about", or "approximately" as used in the present disclosure, unless otherwise specified, means an acceptable error for a particular value as determined by one of ordinary skill in the art, which depends in part on how the value is measured or determined. In certain embodiments, the term "substantially", "about", or "approximately" means within 1, 2, 3, or 4 standard deviations. In certain embodiments, the term "substantially", "about", or "approximately" means within 50%, 20%, 15%, 10%, 9%, 8%, 7%, 6%, 5%, 4%, 3%, 2%, 1%, 0.5%, or 0.05% of a given value or range.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible in light of the above teachings. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

## Claims

1. A buttress applier cartridge (3200, 3300) configured to interface with an end effector (3040) of a surgical stapling instrument, wherein the buttress applier cartridge (3200, 3300) comprises:
a buttress loading zone (3220) configured to interface with an anvil (3240) of the end effector, wherein the buttress loading zone comprises:
a buttress layer (3222, 3314);
a suture (3226, 3316) supporting the buttress layer (3222, 3314); and
a loading assembly (3228, 3324) for securing the suture (3226) to an outer surface of the anvil (3240) as the anvil (3240) approaches the buttress layer (3222, 3314);
**characterized in that** the loading assembly comprises a suture applier rotatable relative to the buttress layer between a resting position and an actuated position, and wherein the suture applier is configured to rotate toward the actuated position as the anvil approaches the buttress layer.

2. The buttress applier cartridge of Claim 1, wherein the anvil comprises a suture grabber (3242, 3336) positioned on the outer surface thereof, and wherein the loading assembly is configured to couple the suture to the suture grabber.

3. The buttress applier cartridge of Claim 1, wherein the suture applier comprises a spring configured to bias the suture applier toward the resting position.

4. The buttress applier cartridge of Claim 1, wherein the suture applier comprises an arm operably coupled to the suture, and wherein the suture is configured to release from the arm as the suture applier rotates toward the resting position after coupling the suture to the anvil.

5. The buttress applier cartridge of Claim 1, wherein the buttress layer comprises an elongate support extending from a surface thereof, wherein the elongate support is configured to couple to the anvil.

6. The buttress applier cartridge of Claim 1, wherein the buttress layer comprises a pin extending from a surface thereof, wherein the pin is configured to couple to the anvil.

7. The buttress applier cartridge of Claim 1, further comprising a cartridge loading zone configured to interface with an elongate channel of the end effector, wherein the cartridge loading zone comprises:
a fastener cartridge removably coupled to the buttress applier cartridge, wherein the fastener cartridge comprises a deck; and
a second buttress layer positioned on the deck.

8. The buttress applier cartridge of Claim 7, wherein the cartridge loading zone comprises a latch configured to support the fastener cartridge, and wherein the latch is configured to release the fastener cartridge as the elongate channel approaches the fastener cartridge.

9. The buttress applier cartridge of any preceding claim, further comprising a housing assembly (3302) including a first leg (3304) and a second leg (3306), the housing assembly (3302) comprising a support platform (3308) positioned between the first leg (3304) and second leg (3306).

10. The buttress applier cartridge of claim 9, wherein the housing assembly is generally U-shaped, defining an open end (3204) and a closed end (3206).

## Patentansprüche

1. Verstärkerapplikatormagazin (3200, 3300), das konfiguriert ist, um mit einem Endeffektor (3040) eines chirurgischen Klammerinstruments eine Schnittstelle auszubilden, wobei das Verstärkerapplikatormagazin (3200, 3300) umfasst:
eine Verstärkerbeladungszone (3220), die konfiguriert ist, um mit einem Amboss (3240) des Endeffektors eine Schnittstelle auszubilden, wobei die Verstärkerbeladungszone umfasst:
eine Verstärkerschicht (3222, 3314);
eine Naht (3226, 3316), die die Verstärkerschicht (3222, 3314) stützt; und
eine Beladungsanordnung (3228, 3324) zum Sichern der Naht (3226) an einer Außenoberfläche des Ambosses (3240), wenn sich der Amboss (3240) der Verstärkerschicht (3222, 3314) nähert;
**dadurch gekennzeichnet, dass** die Beladungsanordnung einen Nahtapplikator umfasst, der relativ zu der Verstärkerschicht zwischen einer Ruheposition und einer betätigten Position drehbar ist, und wobei der Nahtapplikator konfiguriert ist, um sich in Richtung der betätigten Position zu drehen, wenn sich der Amboss der Verstärkerschicht nähert.

2. Verstärkerapplikatormagazin nach Anspruch 1, wobei der Amboss einen Nahtgreifer (3242, 3336) umfasst, der auf der Außenoberfläche davon positioniert ist, und wobei die Beladungsanordnung konfiguriert ist, um die Naht mit dem Nahtgreifer zu koppeln.

3. Verstärkerapplikatormagazin nach Anspruch 1, wobei der Nahtapplikator eine Feder umfasst, die konfiguriert ist, um den Nahtapplikator in Richtung der Ruheposition vorzuspannen.

4. Verstärkerapplikatormagazin nach Anspruch 1, wobei der Nahtapplikator einen Arm umfasst, der mit der Naht wirkgekoppelt ist, und wobei die Naht konfiguriert ist, um sich von dem Arm zu lösen, wenn sich der Nahtapplikator nach dem Koppeln der Naht mit dem Amboss in Richtung der Ruheposition dreht.

5. Verstärkerapplikatormagazin nach Anspruch 1, wobei die Verstärkerschicht eine längliche Stütze umfasst, die sich von einer Oberfläche davon erstreckt, wobei die längliche Stütze konfiguriert ist, um mit dem Amboss zu koppeln.

6. Verstärkerapplikatormagazin nach Anspruch 1, wobei die Verstärkerschicht einen Stift umfasst, der sich von einer Oberfläche davon erstreckt, wobei der Stift konfiguriert ist, um mit dem Amboss zu koppeln.

7. Verstärkerapplikatormagazin nach Anspruch 1, ferner umfassend eine Magazinbeladungszone, die konfiguriert ist, um mit einem länglichen Kanal des Endeffektors eine Schnittstelle auszubilden, wobei die Magazinbeladungszone umfasst:
ein Befestigungselementmagazin, das entfernbar mit dem Verstärkerapplikatormagazin gekoppelt ist, wobei das Befestigungselementmagazin ein Deck umfasst; und
eine zweite Verstärkerschicht, die auf dem Deck positioniert ist.

8. Verstärkerapplikatormagazin nach Anspruch 7, wobei die Magazinbeladungszone eine Verriegelung umfasst, die konfiguriert ist, um das Befestigungselementmagazin zu stützen, und wobei die Verriegelung konfiguriert ist, um das Befestigungselementmagazin zu lösen, wenn sich der längliche Kanal dem Befestigungselementmagazin nähert.

9. Verstärkerapplikatormagazin nach einem der vorstehenden Ansprüche, ferner umfassend eine Gehäuseanordnung (3302), die einen ersten Schenkel (3304) und einen zweiten Schenkel (3306) einschließt, die Gehäuseanordnung (3302) umfassend eine Stützplattform (3308), die zwischen dem ersten Schenkel (3304) und dem zweiten Schenkel (3306) positioniert ist.

10. Verstärkerapplikatormagazin nach Anspruch 9, wobei die Gehäuseanordnung allgemein u-förmig ist und ein offenes Ende (3204) und ein geschlossenes Ende (3206) definiert.

## Revendications

1. Cartouche d'applicateur de contrefort (3200, 3300) conçue pour assurer l'interface avec un effecteur terminal (3040) d'un instrument d'agrafage chirurgical, dans laquelle la cartouche d'applicateur de contrefort (3200, 3300) comprend :
une zone de chargement de contrefort (3220) conçue pour assurer l'interface avec une enclume (3240) de l'effecteur terminal, dans laquelle la zone de chargement de contrefort comprend :
une couche de contrefort (3222, 3314) ;
une suture (3226, 3316) supportant la couche de contrefort (3222, 3314) ; et
un ensemble de chargement (3228, 3324) permettant de fixer la suture (3226) à une surface externe de l'enclume (3240) à mesure que l'enclume (3240) se rapproche de la couche de contrefort (3222, 3314) ;
**caractérisée en ce que** l'ensemble de chargement comprend un applicateur de suture, rotatif par rapport à la couche de contrefort entre une position de repos et une position actionnée, et dans laquelle l'applicateur de suture est conçu pour effectuer une rotation vers la position actionnée à mesure que l'enclume se rapproche de la couche de contrefort.

2. Cartouche d'applicateur de contrefort selon la revendication 1, dans laquelle l'enclume comprend un dispositif de préhension de suture (3242, 3336) positionné sur la surface externe de celle-ci, et dans laquelle l'ensemble de chargement est conçu pour accoupler la suture au dispositif de préhension de suture.

3. Cartouche d'applicateur de contrefort selon la revendication 1, dans laquelle l'applicateur de suture comprend un ressort conçu pour solliciter l'applicateur de suture vers la position de repos.

4. Cartouche d'applicateur de contrefort selon la revendication 1, dans laquelle l'applicateur de suture comprend un bras accouplé fonctionnellement à la suture, et dans laquelle la suture est conçue pour se libérer du bras à mesure que l'applicateur de suture effectue une rotation vers la position de repos après accouplement de la suture à l'enclume.

5. Cartouche d'applicateur de contrefort selon la revendication 1, dans laquelle la couche de contrefort comprend un support allongé s'étendant à partir d'une surface de celle-ci, dans laquelle le support allongé est conçu pour s'accoupler à l'enclume.

6. Cartouche d'applicateur de contrefort selon la revendication 1, dans laquelle la couche de contrefort comprend une goupille s'étendant à partir d'une surface de celle-ci, dans laquelle la goupille est conçue pour s'accoupler à l'enclume.

7. Cartouche d'applicateur de contrefort selon la revendication 1, comprenant en outre une zone de chargement de cartouche conçue pour assurer l'interface avec un canal allongé de l'effecteur terminal, dans laquelle la zone de chargement de cartouche comprend :
une cartouche d'éléments de fixation accouplée de manière amovible à la cartouche d'applicateur de contrefort, dans laquelle la cartouche d'éléments de fixation comprend une platine ; et
une seconde couche de contrefort positionnée sur la platine.

8. Cartouche d'applicateur de contrefort selon la revendication 7, dans laquelle la zone de chargement de cartouche comprend un verrouillage conçu pour supporter la cartouche d'éléments de fixation, et dans laquelle le verrouillage est conçu pour libérer la cartouche d'éléments de fixation à mesure que le canal allongé se rapproche de la cartouche d'éléments de fixation.

9. Cartouche d'applicateur de contrefort selon l'une quelconque revendication précédente, comprenant en outre un ensemble logement (3302) comportant une première branche (3304) et une seconde branche (3306), l'ensemble logement (3302) comprenant une plate-forme de support (3308) positionnée entre la première branche (3304) et la seconde branche (3306).

10. Cartouche d'applicateur de contrefort selon la revendication 9, dans laquelle l'ensemble logement est généralement en forme de U, définissant une extrémité ouverte (3204) et une extrémité fermée (3206).
